# FASCICULE DE BREVET EUROPEEN

(11) **EP 0 986 537 B1**
(45) Date de publication et mention de la délivrance du brevet: **07.08.2002**
(21) Numéro de dépôt: 99910451.6
(22) Date de dépôt: 29.03.1999
(51) Int. Cl.: C07C 391/02

(54) **NOUVEAUX COMPOSES HETEROETHYNYLENES ET COMPOSITIONS PHARMACEUTIQUES ET COSMETIQUES LES CONTENANT**
HETERO-ETHYNYL-VERBINDUNGEN UND DIESE ENTHALTENDE ZUSAMMENSETZUNGEN UND KOSMETIKA
NOVEL HETEROETHYNYLENE COMPOUNDS AND PHARMACEUTICAL AND COSMETIC COMPOSITIONS CONTAINING SAME

(30) Priorité: 31.03.1998 FR 9803977
(43) Date de publication de la demande: 22.03.2000
(73) Titulaire: Galderma Research & Development, S.N.C., 06560 Valbonne (FR)
(72) Inventeur: DIAZ, Philippe, F-06200 Nice (FR); BERNARDON, Jean-Michel, F-06650 Le Rouret (FR)
(74) Mandataire: Andral, Christophe André Louis
(86) Numéro de dépôt international: FR9900727
(87) Numéro de publication internationale: WO9950239

(56) Documents cités:
- EP-A- 0 679 630
- EP-A- 0 776 885
- C. MAIGNAN ET AL: BULL. SOC. CHIM. FR., no. 4, 1986, pages 645-649, XP002114593
- DATABASE CHEMABS [Online] CHEMICAL ABSTRACTS SERVICE, COLUMBUS, OHIO, US STN, CAPLUS, accession no. 1981:550094, XP002114599 & E.Y. KOLOSOV ET AL: ZH.ORG. KHIM., vol. 17, no. 6, 1981, pages 1184-1190,
- DATABASE CHEMABS [Online] CHEMICAL ABSTRACTS SERVICE, COLUMBUS, OHIO, US STN, CAPLUS, accession no. 1976:523496, XP002114600 & L.A. BEZ'YAZYCHNAYA ET AL: ZH. OBSHCH. KHIM., vol. 46, no. 7, 1976, pages 1557-1566,
- R.S. PALEY ET AL: J. ORG. CHEM., vol. 62, no. 18, 1997, pages 6326-6343, XP002114594
- X. HUANG ET AL: SYNTHESIS, no. 10, 1996, pages 1191-1192, XP002114595
- G. SOLLADI ET AL: SYNTHESIS, no. 11, 1991, pages 1011-1012, XP002114596
- J. BARLUENGA ET AL: J. CHEM. SOC. PERKIN TRANS 1, no. 12, 1987, pages 2605-2609, XP002114597
- M.C. BARNABEU ET AL: TETRAHEDRON LETT., vol. 36, no. 22, 1995, pages 3901-3904, XP002114598
- M.C. BERNABEU ET AL: TETRAHEDRON LETT., vol. 37, no. 20, 1996, pages 3595-3598, XP004029356
- E. ARCE ET AL: TETRAHEDRON: ASYMMETRY, vol. 6, no. 7, 1995, pages 1757-1764, XP004048152
- DATABASE CHEMABS [Online] CHEMICAL ABSTRACTS SERVICE, COLUMBUS, OHIO, US STN, CAPLUS, accession no. 1972:501313, XP002114939 & CHEMICAL ABSTRACTS, vol. 77, no. 15, 9 octobre 1972 (1972-10-09) Columbus, Ohio, US; abstract no. 101313z, & E. SCHMID ET AL: HELV. CHIM. ACTA, vol. 55, no. 5, 1972, pages 1625-1674,

## Description

L'invention concerne, à titre de produits industriels nouveaux et utiles, des composés hétéroéthynylénés. Elle concerne également l'utilisation de ces nouveaux composés dans des compositions pharmaceutiques destinées à un usage en médecine humaine ou vétérinaire, ou bien encore dans des compositions cosmétiques.

La Damanderesse avait déjà proposé dans la demande de brevet EP 0 679 630 des composés bicycliques aromatiques et dans la demande de brevet EP 0 0776 885 des composés biaromatiques portant un groupement adamantyl en ortho, l'ensemble de ces composés présentant une activité agoniste ou antagoniste des récepteurs RAR et pouvant être utilisés en médecine humaine ou vétérinaire ou dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante : 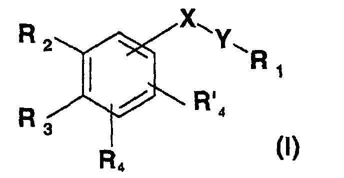 dans laquelle:
- R₁ représente :
   (i) le radical -CH3
   (ii) le radical -CH2-O-R₅
   (iii) le radical -COR₆
   R₅ et R₆ ayant les significations données ci-après,
- X représente : O, Se , S(O)n, n étant 0, 1 ou 2,
- Y représente un radical divalent qui a pour formule :
   a) 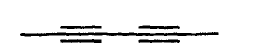
   b) 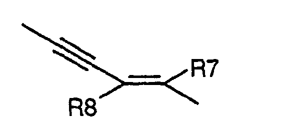
   R₇, et R₈ ayant les significations données ci-après,
- R₂ et R₃ pris ensemble forment avec le cycle aromatique adjacent un cycle à 5 ou 6 chainons éventuellement substitué par des groupes méthyle et / ou éventuellement interrompu par un atome d'oxygène ou de soufre,
   sachant que- R₂ et R₃ indépendamment identiques ou différents- peuvent représenter :
   a) un atome d'hydrogène,
   b) un radical choisi parmi les radicaux
      - méthyl
      - tertiobutyl,
      - 1-méthylcyclohexyl,
      - 1-Adamantyl,
   c) un radical -OR₉
   d) un radical polyéther
   e) un atome d'halogène
   R₉ ayant la signification donnée ci-après,
   étant entendu que au moins un des substituants- R₂ et R₃ représente (b),
- R₄ représente un atome d'hydrogène, un radical alkyle de 1 à 6 atomes de carbone, un radical OR₁₀, un radical polyéther un radical COR₁₁, ou un atome d'halogène,
   R₁₀ et R₁₁ ayant les significations données ci-après,
- R'₄ représente un atome d'hydrogène ou un atome d'halogène,
- R₅ représente un atome d'hydrogène ou un radical alkyle de 1 à 6 atomes de carbone,
- R₆ représente :
   (i) un atome d'hydrogène
   (ii) un radical alkyle de 1 à 6 atomes de carbone
   (iii) le radical OR₁₂
      R₁₂ ayant la signification donnée ci-après,
   (iv) le radical de formule 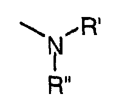 R' et R" ayant les significations données ci-après,
- R₇, et R₈, identiques ou différents, représentent un atome d'hydrogène un radical alkyle de 1 à 6 atomes de carbone, ou un radical aryle,
- -R₉ et R₁₀, identiques ou différents, représentent un atome d'hydrogène, un radical alkyle de 1 à 6 atomes de carbone, un radical aryle éventuellement substitué, un radical aralkyle éventuellement substitué, un radical monohydroxyalkyle ou polyhydroxyalkyle ou un radical acyle de 1 à 4 atomes de carbone,
   R₁₁ représente un radical alkyle inférieur, un radical OR₁₃ ou un radical 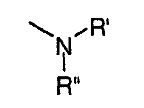 R₁₃, R' et R" ayant les significations données ci-après,
- R₁₂ et R₁₃ identiques ou différents représentent un atome d'hydrogène, un radical alkyle de 1 à 6 atomes de carbone, un radical aryle, un radical aralkyle éventuellement substitué, un radical monohydroxyalkyle ou un radical polyhydroxyalkyle,
- R' et R", identiques ou différents, représentent un atome d'hydrogène, un radical alkyle de 1 à 6 atomes de carbone, un radical mono ou polyhydroxyalkyle, un radical aryle éventuellement substitué ou un reste d'amino acide, ou encore pris ensemble avec l'atome d'azote forment un hétérocycle,

L'invention vise également les sels des composés de formule (I) lorsque R₁ représente une fonction acide carboxylique et les isomères géométriques et optiques desdits composés de formule (I).

Lorsque les composés selon l'invention se présentent sous forme de sels, il s'agit de préférence de sels d'un métal alcalin ou alcalino-terreux, ou encore de zinc ou d'une amine organique.

Par radical alkyle de 1 à 6 atomes de carboné on entend de préférence les radicaux méthyle, éthyle, isopropyle, butyle et tertiobutyle.

Par radical monohydroxyalkyle, on doit entendre un radical ayant de 1 à 6 atomes de carbone, notamment un radical 2-hydroxyéthyle, 2-hydroxypropyle ou 3-hydroxypropyle.

Par radical polyhydroxyalkyle, on doit entendre un radical contenant de 2 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle éventuellement substitué, on doit entendre un radical phényle éventuellement substitué par au moins un atome d'halogène, un hydroxyle ou une fonction nitro.

Par radical aralkyle éventuellement substitué, on doit entendre le radical benzyle ou phénéthyle éventuellement substitué par au moins un atome d'halogène, un hydroxyle ou une fonction nitro.

Par radical acyle de 1 à 4 atomes de carbone on entend en particulier un radical acétyle ou propionyle.

Par reste d'aminoacide on doit entendre un reste dérivant par exemple de l'un des 20 aminoacides de configuration L ou D constitutifs de protéines de mammifères.

Par hétérocycle, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Par radical polyéther, on entend un radical ayant de 1 à 6 atomes de carbone et de 1 à 3 atomes d'oxygène ou de soufre, tels que les radicaux méthoxyméthyl éther, méthoxyéthoxyméthyl éther ou méthylthiométhyl éther.

Par atome d'halogène, on entend de préférence un atome de fluor, de brome ou de chlore.

Parmi les composés de formule (I) ci-dessus rentrant dans le cadre de la présente invention, on peut notamment citer les suivants :
3-Methyl-5-(5,5,8,8-tetramethyl-5,6,7,8-tetrahydro-naphtalen-2-ylselanyl)-pent-2-en-4-ynoate d'éthyle
Acide-3-méthyl-5-(5,5,8,8-tetraméthyl-5,6,7,8,-tetrahydro-naphtalen-2-ylselanyl)-pent-2-en-4-ynoique
3-méthyl-5-(3,5,5,8,8-pentaméthyl-5,6,7,8-tetrahydro-naphtalen-2-ylselanyl)-pent-2-en-4-ynoate d'éthyle
3-méthyl-5-(5,5,8,8-tetraméthyl-5,6,7,8-tetrahydro-naphtalen-2-ylsulfanyl)-pent-2-en-4-ynoate d'éthyle
Acide 3-méthyl-5-(5,5,8,8-tetraméthyl-5,6,7,8-tetrahydro-naphtalen-2-ylsulfanyl)-pent-2-en-4-ynoique
3-méthyl-5-(3,5,5,8,8-pentaméthyl-5,6,7,8-tetrahydro-naphtalen-2-ylsulfanyl)-pent-2-en-4-ynoate d'éthyle
Acide 3-méthyl-5-(3,5,5,8,8-pentaméthyl-5,6,7,8-tetrahydro-naphtalen-2-ylsulfanyl)-pent-2-en-4-ynoique
3-Methyl-5-(5,5,8,8-tetramethyl-3-propoxy-5,6,7,8-tetrahydro-naphthalen-2-ylselanyl)-pent-2-en-4-ynoate d'éthyle
Acide 3-Methyl-5-(5,5,8,8-tetramethyl-3-propoxy-5,6,7,8-tetrahydro-naphthalen-2-ylselanyl)-pent-2-en-4-ynoïque.
3-Methyl-5-(5,5,8,8-tetramethyl-4-propoxy-5,6,7,8-tetrahydro-naphthalen-2-ylselanyl)-pent-2-en-4-ynoate d'éthyle
Acide 3-Methyl-5-(5,5,8,8-tetramethyl-4-propoxy-5,6,7,8-tetrahydro-naphthalen-2-ylselanyl)-pent-2-en-4-ynoïque
5-(4-Methoxymethoxy-5,5,8,8-tetramethyl-5,6,7,8-tetrahydro-naphthalen-2-ylselanyl)-3-methyl-pent-2-en-4-ynoate d'éthyle
Acide 5-(4-Methoxymethoxy-5,5,8,8-tetramethyl-5,6,7,8-tetrahydro-naphthalen-2-ylselanyl)-3-methyl-pent-2-en-4-ynoïque
5-(3-Hydroxy-5,5,8,8-tetramethyl-5,6,7,8-tetrahydro-naphthalen-2-ylselanyl)-3-methyl-pent-2-en-4-ynoate d'éthyle
Acide 5-(3,5,5,8,8-Pentamethyl-5,6,7,8-tetrahydro-naphthalen-2-ylselanyl)-3-phenyl-pent-2-en-4-ynoïque
Acide 5-(4-Methoxymethoxy-5,5,8,8-tetramethyl-5,6,7,8-tetrahydro-naphthalen-2-ylselanyl)-3-phenyl-pent-2-en-4-ynoïque
5-(4-Hydroxy-5,5,8,8-tetramethyl-5,6,7,8-tetrahydro-naphthalen-2-ylselanyl)-3-phenyl-pent-2-en-4-ynoate de méthyle
Acide 3-propyl-5-(5,5,8,8-tetraméthyl-5,6,7,8,-tetrahydro-naphtalen-2-ylselanyl)-pent-2-en-4-ynoique
Acide 5-(4-Benzyloxy-3-bromo-5,5,8,8-tetramethyl-5,6,7,8-tetrahydro-naphthalen-2-ylselanyl)-3-methyl-pent-2-en-4-ynoïque
Acide 5-(4-Benzyloxy-5,5,8,8-tetramethyl-5,6,7,8-tetrahydro-naphthalen-2-ylselanyl)-3-phenyl-pent-2-en-4-ynoïque
5-[5-Adamantan-1-yl-4-(2-methoxy-ethoxymethoxy)-2-methyl-phenylselanyl]-3-methyl-pent-2-en-4-ynoate d'éthyle
Acide 5-[5-Adamantan-1-yl-4-(2-methoxy-ethoxymethoxy)-2-methyl-phenylselanyl]-3-methyl-pent-2-en-4-ynoïque
Acide 5-[5-Adamantan-1-yl-4-(2-methoxy-ethoxymethoxy)-2-methyl-phenylselanyl]-3-propyl-pent-2-en-4-ynoïque
Acide 5-[5-Adamantan-1-yl-4-(2-methoxy-ethoxymethoxy)-2-methyl-phenylselanyl]-3-phenyl-pent-2-en-4-ynoïque
5-(3,5-Di-tert-butyl-2-methoxymethoxy-phenylselanyl)-3-methyl-pent-2-en-4-ynoate d'éthyle
Acide 5-(3,5-Di-*tert*-butyl-2-methoxymethoxy-phenylselanyl)-3-methyl-pent-2-en-4-ynoïque
3-Methyl-5-(5,5,8,8-tetramethyl-3-hexyloxy-5,6,7,8-tetrahydro-naphthaien-2-ylselanyl)-pent-2-en-4-ynoate d' éthyle
Acide 3-Methyl-5-(5,5,8,8-tetramethyl-3-hexyloxy-5,6,7,8-tetrahydro-naphthalen-2-ylselanyl)-pent-2-en-4-ynoïque
Acide 5-[4-Adamantan-1-yl-3-(2-methoxy-ethoxymethoxy)-phenylselanyl]-3-propyl-pent-2-en-4-ynoïque
5-[2-(adamantan-1-yl)-1-méthoxyéthoxyméthoxyphen-5-ylselanyl)-3-methyl-pent-2-en-4-ynoate d'éthyle
5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoate d'éthyle
Acide (Z)-5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoïque
Acide (E)-5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoïque
5-(3,5,5,8,8-Pentamethyl-5,6,7,8-tetrahydro-naphthalen-2-ylsulfanyl)-penta-2,4-diynoate de méthyle
5-(3-Benzyloxy-5,5,8,8-tetramethyl-5,8,7,8-tetrahydro-naphthalen-2-ylselanyl)-3-methyl-pent-2-en-4-ynoate d'ethyle
Acide 5-(3-Benzyloxy-5,5,8,8-tetramethyl-5,6,7,8-tetrahydro-naphthalen-2-ylselanyl)-3-methyl-pent-2-en-4-ynoïque
5-[3-(2-methoxyethoxymethoxy)-5,5,8,8-tetramethyl-5,6,7,8-tetrahydro-naphthalen-2-ylselanyl]-3-methyl-pent-2-en-4-ynoate d'ethyle
Acide 5-[3-(2-methoxyethoxymethoxy)-5,5,8,8-tetramethyl-5,6,7,8-tetrahydro-naphthalen-2-ylselanyl]-3-methyl-pent-2-en-4-ynoïque
5-(3,5,5,8,8-Pentamethyl-5,6,7,8-tetrahydro-naphthalen-2-ylselanyl)-penta-2,4-diynoate de méthyle
5-(4-Benzyloxy-5,5,8,8-tetramethyl-5,6,7,8-tetrahydro-naphthalen-2-ylselanyl)-3-methyl-pent-2-en-4-ynoate d'ethyle
5-[3-(5-Hydroxy-pentyloxy)-5,5,8,8-tetramethyl-5,6,7,8-tetrahydro-naphthalen-2-ylselanyl]-3-methyl-pent-2-en-4-ynoate d'éthyle
Acide 5-(4-Fluoro-3-methyl-phenylselanyl)-3-methyl-pent-2-en-4-ynoïque
Acide 3-Methyl-5-p-tolylselanyl-pent-2-en-4-ynoïque
Acide 5-(6-bromo-4,4-Dimethyl-thiochroman-8-ylselanyl)-3-methyl-pent-2-en-4-ynoique

Selon la présente invention les composés de formule (I) plus particulièrement préférés sont ceux pour lesquels au moins l'une des, et de préférence toutes les, conditions suivantes est remplie :
- R₁ représente le radical -COR₆;
- X représente le radical Se ou S;
- le groupement -X-Y-R1 est en position para par rapport au substituant R3 sur le cycle aromatique;
- R₂ et R₃ pris ensemble forment avec le cycle aromatique adjacent un cycle à 5 ou 6 chaînons éventuellement substitué par des groupes méthyle et / ou éventuellement interrompu par un atome d'oxygène ou de soufre, ou R₂ ou R₃ est un radical 1-Adamantyl.

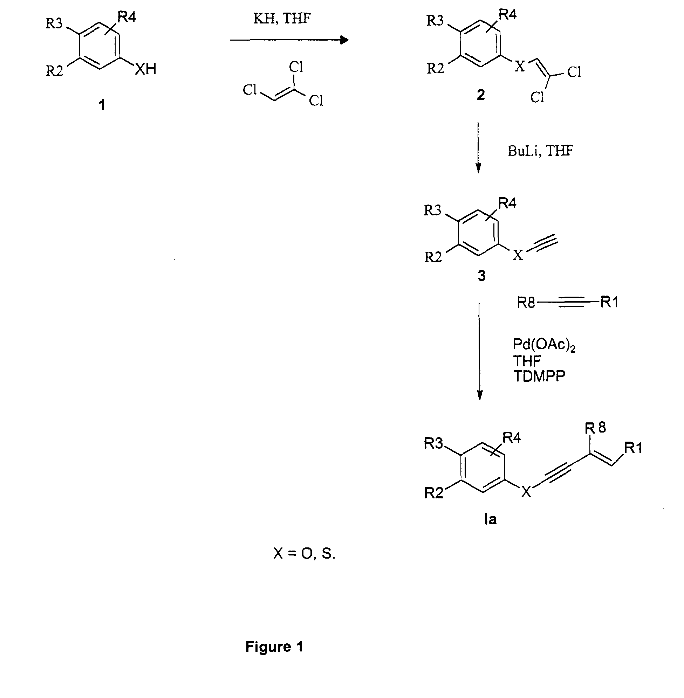

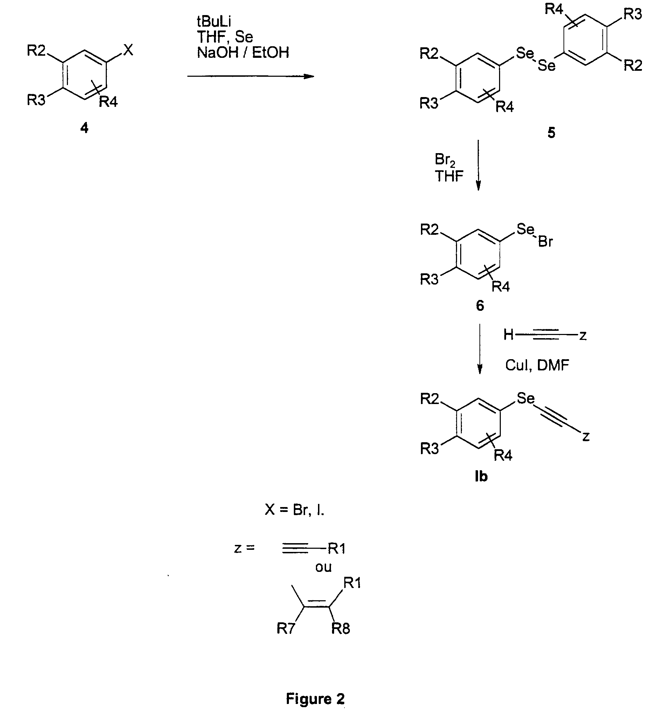

La présente invention a également pour objet les procédés de préparation des composés de formule (I), en particulier selon les schémas réactionnels donnés aux figures 1 et 2.

En se référant à la figure 1, les dérivés de formule **(la)** peuvent être préparés par une suite de réactions comprenant, l'action d'une base telle que l'hydrure de potassium ou de sodium sur le produit **(1)**, suivie du couplage avec le trichloroéthylène. Le produit **(2)** obtenu est soumis à l'action d'une base lithiée telle que BuLi dans un solvant comme le THF. L'acétylénique **(3)** obtenu peut être couplé avec un alcyne disubstitué, en présence d'un catalyseur au palladium.

En se référant à la figure 2, les dérivés de formule **(Ib)** peuvent être préparés par une suite de réactions comprenant, l'action d'une base lithiée telle que le tBuLi sur le produit **(4)** dans un solvant comme le THF, suivie de l'addition de selenium et de la formation du dimère par oxydation en milieu basique (EtOH, NaOH). Le produit **(5)** obtenu est soumis à l'action de brome dans un solvant comme le THF puis couplé avec un acétylénique vrai en présence d'un catalyseur au cuivre.

Le produit **(la)** avec Y différent d'un atome d'oxygène, peut être oxydé en sulfone ou sulfoxyde par action d'oxydant tel que l'acide meta perbenzoïque ou le periodate de sodium.

Lorsque R₁ représente le radical COOH, les composés sont préparés en protégeant R₁ par un groupe protecteur de type alkyle. La saponification de la fonction ester en présence d'une base, telle l'hydroxyde de sodium ou de lithium dans un solvant alcoolique ou dans le THF conduit aux acides correspondants.

Lorsque R₁ représente un radical alcool, les composés peuvent être obtenus à partir de l'acide par réduction en présence d'hydrure comme l'hydrure de bore. L'alcool peut être ethérifié selon les méthode classiques.

Lorsque R₁ représente un radical aldéhyde, les composés peuvent être obtenus par oxydation des alcools correspondants par action d'oxyde de manganèse ou de pyridinium dichromate.

Lorsque R₁ représente un radical amide, les composés peuvent être obtenus par transformation de l'acide en chlorure d'acide puis par réaction avec une amine appropriée.

Ces composés se lient aux récepteurs RXRs, certains possédant une activité agoniste, d'autres une activité antagoniste. Certains de ces composés peuvent se lier aussi aux récepteurs RARs.

Les propriétés de binding et de transactivation comme agoniste aux récepteurs RXRs peuvent être déterminées par des méthodes connues dans l'art, comme par exemple : LEVIN et al, Nature 1992, **355**, 359-61 ; ALLENBY et al, Proc. Natl. Acad. Sci., 1993, **90**, 30-4.

L'activité agoniste RXRs peut être aussi déterminée par le test tel que décrit dans la demande de brevet français n° 95-07301 déposée le 19 juin 1995 par la demanderesse. Ce test comprend les étapes suivantes : (i) on applique topiquement sur une partie de la peau d'un mammifère une quantité suffisante d'un composé qui est un ligand actif d'au moins un récepteur de la superfamille des récepteurs nucléaires stéroïdiens/thyroïdiens autre qu'un ligand spécifique des récepteurs RXRs et pouvant s'hétérodimériser avec les RXRs tel qu'une molécule agoniste des RARs, (ii) on administre par voie systémique ou topique sur cette même partie de la peau du mammifère avant, pendant ou après l'étape (i) une molécule susceptible de présenter une activité agoniste des RXRs, (iii) on évalue la réponse sur la partie de la peau ainsi traitée du mammifère. Ainsi la réponse à une application topique sur l'oreille d'un mammifère d'une molécule agoniste des RARs qui correspond à une augmentation de l'épaisseur de cette oreille peut être augmentée par l'administration par voie systémique ou topique d'une molécule agoniste des récepteurs RXRs.

L'activité antagoniste RXRa peut être évaluée dans le test de transactivation par détermination de la dose (IC₅₀) qui inhibe de 50% l'activité transactivatrice d'un agoniste sélectif RXRa: l'acide 6-[1-(3,5,5,8,8-pentaméthyl-5,6,7,8-tétrahydronaphtalen-2-yl)cyclopropyl]nicotinique (CD 3127) selon le protocole suivant :
Les cellules Hela sont co-transfectées avec un vecteur d'expression codant pour RXRa (p565-RXRa) et un plasmide rapporteur contenant l'élément de réponse 1/2 CRBP II cloné en amont du promoteur hétérologue de la thymidine kinase et du gène rapporteur de la chloramphènicolm-acétyl-transfèrase (CAT). Dix-huit heures après co-transfection les cellules sont traitées avec une concentration fixe du CD 3127 et des concentrations croissantes de la molécule à évaluer. Après vingt-quatre heures de traitement le dosage de l'activité CAT est effectué par ELISA. La concentration fixe de CD3127 utilisée est 10⁻⁸M et correspond à son EC₅₀.

La présente invention a également pour objet à titre de médicament les composés de formule (I) telle que définie ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique, ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation, pour prévenir ou pour réparer les vergetures, ou encore pour favoriser la cicatrisation,
10) pour lutter contre les troubles de la fonction sébacée tels que l'hyperséborrhée de l'acné ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose, l'hypertension, le diabète non-insulino dépendant ainsi que l'obésité,
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des a-hydroxy ou a-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃.

Par anti-radicaux libres, on entend par exemple l'a-tocophérol, la Super Oxyde Dismutate, l'Ubiquinol ou certains chélatants de métaux. Par a-hydroxy ou a-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3- méthyl 1,2,4-benzothiadiazine 1,1-dioxyde) et le Phénytoïn (5,4-diphényl-imidazolidine 2,4-dione); des agents anti-inflammatoires non stéroïdiens; des caroténoïdes et, notamment, le b-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés. Dans ce qui suit ou ce qui précède, les pourcentages sont donnés en poids sauf mention contraire.

### EXEMPLE 1 :

### 3-Methyl-5-(5,5,8,8-tetramethyl-5,6,7,8-tetrahydro-naphtalen-2-ylselanyl)-pent-2-en-4-ynoate d'ethyle

### (a) 5,6,7,8-tetrahydro-5,5,8,8-tetramethylnaphtalene-2-disélénide

Une solution de tert-butyllithium 1,7 M dans le pentane (37,4 mmol, 22 ml) est additionnée à une solution de 2-bromo-5,6,7,8-tetrahydro-5,5,8,8-tetraméthylnaphtalene (4,22 g, 15,8 mmol) dans le THF (100 ml) à -78°C en 10 min. Le mélange est agité à 0°C 30 min. Le sélénium(1,33 g, 16,8 mmol) est additionné en 2 fois. Le mélange est agité à 0°C 15 min, puis à température ambiante 30 min. Une solution d'HCl 1N (40 ml) est additionnée, puis le mélange réactionnel est traité par de l'éther éthylique. La phase organique est lavée 2 fois à l'eau, séchée sur sulfate de magnésium anhydre et concentrée à l'évaporateur rotatif sous vide à 40°C. 10 ml d'éthanol et 50 mg de soude sont additionnés à l'huile obtenue. Le mélange est agité vigoureusement quelques minutes à l'air (qsp tout précipite), puis est concentré à l'évaporateur rotatif sous vide à 40°C. Le solide obtenu est filtré sur silice (élution heptane) puis cristallisé dans un mélange éthanol / éther.
Solide orange. Masse : 2,9 g. Rendement : 69%.
RMN 1H (CDCl₃) : 1,21 (6H, s), 1,25 (6H, s), 1,65 (4H, s), 7,20 (1H Ar, d, J=8,25 Hz), 7,38 (1H Ar, dd ,J=1,9 Hz, J=8,25 Hz), 7,51 (1H Ar, d, J=1,9 Hz).

### (b) 3-méthyl-pent-2-en-4-ynoate d'éthyle

Du KF (58 mg, 1 mmol) est additionné à une solution de 3-méthyl-5-triméthylsilanyl-pent-2-en-4-ynoate d'éthyle (J. Am. Chem. Soc. 1997, 119, 698-708), (210 mg, 1 mmol) dans 10 ml de méthanol, 10 ml de THF et 1 ml d'eau. Le mélange est agité 8 h à température ambiante. Il est ensuite traité par de l'éther éthylique. La phase organique est lavée plusieurs fois à l'eau, séchée sur sulfate de magnésium anhydre et concentrée.
Colonne SiO₂ (dichlorométhane 30 / heptane 70).
Huile. Masse : 100 mg. Rendement 72%.
RMN 1 H (CDCl₃): 0,00 (9H, s), 1,05-1,08 (3H, t), 2,06 (3H, s), 3,91-4,00 (2H, q), 5,88-5,89 (1H, m).

### (c) 3-méthyl-5-(5,5,8,8-tetraméthyl-5,6,7,8,-tetrahydro-naphtalen-2-ylselanyl)-pent-2-en-4-ynoate d'éthyle

Du brome (0,048 ml, 0,93 mmol), est additionné à une solution de 5,6,7,8-tétrahydro-5,5,8,8-tétraméthylnaphtalene-2-disélénide (500 mg, 0,94 mmol) dans le THF (1 ml). Le mélange est agité à température ambiante 2 h, puis le solvant est éliminé par un fort courant d'azote. De l'iodure de cuivre (Cul) (715 mg, 3,75 mmol), du 3-méthyl-pent-2-en-4-ynoate d'éthyle (232 mg, 1,67 mmol), et du DMF (5 ml), sont additionnés. Le mélange réactionnel est agité à température ambiante 3 h puis est traité par de l'éther éthylique et une solution d'ammoniaque. La phase organique est lavée 2 fois à l'eau, séchée sur sulfate de magnésium anhydre et concentrée à l'évaporateur rotatif sous vide à 40°C.
Colonne SiO₂ (heptane 70 / dichlorométhane 30)
huile incolore. Masse 500 mg. Rendement : 74%.
RMN 1H (CDCl3) : 1,27 (6H, s), 1,28 (6H, s), 1,25-1,31 (3H, t), 1,68 (4H, s), 2,34 (3H, d), 4,12-4,21 (2H, q), 6,01-6,02 (1H, d), 7,27-7,28 (2H Ar, d), 7,45 (1H Ar, s).

### EXEMPLE 2 :

### Acide 3-méthyl-5-(5,5,8,8-tetraméthyl-5,6,7,8,-tetrahydro-naphtalen-2-ylselanyl)-pent-2-en-4-ynoique

De l'hydroxyde de lithium (500 mg) est additionné à la solution de 3-méthyl-5-(5,5,8,8-tetraméthyl-5,6,7,8,-tetrahydro-naphtalen-2-ylselanyl)-pent-2-en-4-ynoate d'éthyle (500 mg, 1,24 mmol), dans 10 ml de THF. 2 ml d'un mélange eau / méthanol (1/1) sont ajoutés. Le milieu réactionnel est chauffé 8 h à reflux. Il est ensuite versé sur un mélange éther éthylique / eau, acidifié à pH 1 par une solution d'acide chlorhydrique concentré et extrait à l'éther éthylique. Après décantation, la phase organique est lavée 2 fois à l'eau, séchée sur sulfate de magnésium anhydre et concentrée à l'évaporateur rotatif sous vide à 40°C.
Poudre jaunatre. Masse 270 mg. Rendement 58%. Tf : 130°C.
RMN1H (CDCl₃) : 1,27 (6H, s), 1,28 (6H,s), 1,68 (4H, s), 2,35 (3H, d), 6,03 (1H, d), 7,28 (2H Ar, s), 7,45 (1H Ar, s).

### EXEMPLE 3 :

### 3-méthyl-5-(3,5,5,8,8-pentaméthyl-5,6,7,8-tetrahydro-naphtalen-2-ylselanyl)-pent-2-en-4-ynoate d'éthyle

### (a) 5,6,7,8-tetrahydro-3,5,5,8,8-pentaméthylnaphtalene-2-disélénide.

De manière analogue à l'exemple 1(a), par réaction de 4,4 g (15,8 mmol) de 2-bromo-(5,6,7,8-tetrahydro-3,5,5,8,8-pentaméthylnaphtalene), avec 22 ml de tert-Butyllithium et du Sélénium (1,33 g, 16,8 mmol) dans 100 ml de THF, on obtient 3,26 g (74%) du dérivé sélénié attendu sous forme d'un solide jaune. (Tf : 126°C).
RMN 1H (CDCl₃) : 1,14 (6H, s), 1,23 (6H, s), 1,61 (4H, s), 2,35 (3H, s), 7,05 (1H Ar, s), 7,55 (1H Ar, s).

### (b) 3-méthyl-5-(3,5,5,8,8-pentaméthyl-5,6,7,8-tetrahydro-naphtalen-2-ylselanyl)-pent-2-en-4-ynoate d'éthyle

De manière analogue à l'exemple 1(c), par réaction de 530 mg (0,94 mmol) de 5,6,7,8-tetrahydro-3,5,5,8,8-pentaméthylnaphtalene-2-disélénide)dans 1 ml de THF,
avec du brome (0,048 ml, 0,93 mmol), de l'iodure de cuivre (715 mg, 3,75 mmol),du 3-méthyl-pent-2-en-4-ynoate d'éthyle (232 mg, 1,68 mmol) dans 5 ml de DMF, on obtient 690 mg (88%) du dérivé ester attendu sous forme d'huile.
RMN1H (CDCl₃) : 1,26 (6H, s), 1,29 (6H, s), 1,25-1,31 (3H, t), 1,67 (4H, s), 2,31 (3H, s), 2,35-2,36 (3H, d), 4,12-4,21 (2H, q), 6,02-6,03 (1H, d), 7,10 (1H Ar, s), 7,62 (1H Ar, s).

### EXEMPLE 4 :

### Acide 3-méthyl-5-(3,5,5,8,8-pentaméthyl-5,6,7,8-tetrahydro-naphtalen-2-ylselanyl)-pent-2-en-4-ynoique

De manière analogue à l'exemple 2, par réaction de 600 mg de 3-méthyl-5-(3,5,5,8,8-pentaméthyl-5,6,7,8-tetrahydro-naphtaten-2-ylselanyl)-pent-2-en-4-ynoate d'éthyle avec 600 mg de LiOH dans 15 ml de THF, on obtient 410 mg (73%) d'une poudre jaune. Tf :175°C.
RMN1H (CDCl₃) : 1,26 (6H, s), 1,29 (6H, s), 1,67 (4H, s), 2,32 (3H, s), 2,36-2,37 (3H, d), 6,04 (1H, d), 7,10 (1H Ar, s), 7,62 (1H Ar, s).

### EXEMPLE 5 :

### 3-méthyl-5-(5,5,8,8-tetraméthyl-5,6,7,8-tetrahydro-naphtalen-2-ylsulfanyl)-pent-2-en-4-ynoate d'éthyle

### (a) 6-(2,2-dichloro-vinylsulfanyl)-1,1,4,4-tetraméthyl-1,2,3,4-tetrahydro-naphtalène

A une suspension d'hydrure de potassium 35% (19,77 g), dans 300 ml de THF, on ajoute 5,6,7,8-tetrahydro-5,5,8,8-tetraméthylnaphtalene-2-disulfure (J. Med. Chem. 1995, 38, 3171) (17,5 g, 79,5 mol), dans 100 ml de THF. Le mélange est agité 1 h à température ambiante puis refroidit à -50°C. On y ajoute ensuite goutte à goutte une solution de trichloroéthylène (7,9 ml) dans 100 ml de THF. On laisse remonter jusqu'à température ambiante et agite 1 h. Le mélange est ensuite concentré puis traité par de l'éther éthylique. La phase organique est lavée plusieurs fois à l'eau, séchée sur sulfate de magnésium anhydre et concentrée.
Huile brune. Masse : 25 g. Rendement : 100%.
RMN 1H (CDCl₃) : 1,17 (6H, s), 1,18 (6H, s), 1,58 (4H,s), 6,41 (1H, s), 7,06-7,10 (1H Ar, dd, J=2,04 Hz, J'=6,2 Hz), 7,17-7,20 (1H Ar, d, J=8,25 Hz), 7,26-7,27 (1H Ar, d, J=2 Hz).

### (b) 6-éthynylsulfanyl-1,1,4,4-tetraméthyl-1,2,3,4-tetrahydro-naphtalène

Une solution de 6-(2,2-dichloro-vinylsulfanyl)-1,1,4,4-tetraméthyl-1,2,3,4-tetrahydro-naphtalène (7 g; 22,2 mmol) dans 70 ml de THF est refroidie à -70°C puis traitée avec du n Butyllithium 2,5M/Hexane (19,5 ml ; 48,8 mmol). On laisse remonter à température ambiante et agiter 3 h. Le mélange est concentré puis traité par de l'éther éthylique. La phase organique est lavée plusieurs fois à l'eau, séchée sur sulfate de magnésium anhydre et concentrée à l'évaporateur rotatif sous vide à 40°C.
Colonne SiO₂ (Heptane)
Huile jaune. Masse 3,4 g. Rendement 63%.
RMN 1H (CDCl₃): 1,26 (6H, s), 1,27 (6H, s), 1,67 (4H, s), 3,19 (1H, s), 7,20-7,24 (1H Ar, dd, J=2,02 Hz, J'=6,33 Hz), 7,27-7,31 (1H Ar, d, J=8,32 Hz), 7,35-7,36 (1H Ar, d, J=2 Hz).

### (c) 3-méthyl-5-(5,5,8,8-tetraméthyl-5,6,7,8-tetrahydro-naphtalen-2-ylsulfanyl)-pent-2-en-4-ynoate d'éthyle

De manière analogue à J. Am. Chem. Soc. 1997,119,698-708, par réaction de Pd(OAc)2 (22 mg, 0,098 mmol), tris (2,6-diméthoxyphényl) phosphine [TDMPP] (44 mg), d'éthyl-2-butynoate (650 mg, 5,8 mmol), avec 6-éthynylsulfanyl-1,1,4,4-tetraméthyl-1,2,3,4-tetrahydro-naphtalène (800 mg, 3,27 mmol), dans 18 ml de toluène, on obtient 603 mg (52%) de produit attendu sous forme d'huile jaune.
RMN 1H (CDCl₃) : 1,26 (6H, s), 1,28 (6H, s), 1,25-1,31 (3H, t), 1,67 (4H, s), 2,36 (3H, d), 4,13- 4,21 (2H, q), 6,00-6,02 (1H, d), 7,16-7,20 (1H Ar, dd, J=2,12 Hz, J'=6,27 Hz), 7,29-7,32 (1H Ar, d, J=8,35 Hz), 7,34-7,35 (1H Ar, d, J=2,1 Hz).

### EXEMPLE 6 :

### Acide 3-méthyl-5-(5,5,8,8-tetraméthyl-5,6,7,8-tetrahydro-naphtalen-2-ylsulfanyl)-pent-2-en-4-ynoique

A une solution de 3-méthyl-5-(5,5,8,8-tetraméthyl-5,6,7,8-tetrahydro-naphtalen-2-ylsulfanyl)-pent-2-en-4-ynoate d'éthyle (605 mg, 1,7 mmol) dans 15 ml de THF est additionné NaOH (100 mg, 2,5 mmol) dans de l'eau. Le milieu est alors chauffé 6 h à reflux. Il est ensuite versé sur un mélange éther éthylique / eau, acidifié à pH 1 par une solution d'acide chlorhydrique concentré et extrait à l'éther éthylique. Après décantation, la phase organique est lavée 2 fois à l'eau, séchée sur sulfate de magnésium anhydre et concentrée à l'évaporateur rotatif sous vide à 40°C. Le résidu est ensuite cristallisé dans du méthanol.
Poudre jaunâtre. Masse 323 mg. Rendement 58%. Tf : 134°C.
RMN1H (CDCl₃) : 1,27 (6H, s), 1,28 (6H, s), 1,68 (4H, s), 2,37-2,38 (3H, d), 6,02-6,03 (1H, d), 7,17-7,21 (1H Ar, dd, J=2,08 Hz, J'=6,3 Hz), 7,30-7,33 (1H Ar, d, J=8,38 Hz), 7,35-7,36 (1 H Ar, d, J=2,04 Hz).

### EXEMPLE 7 :

### 3-méthyl-5-(3,5,5,8,8-pentaméthyl-5,6,7,8-tetrahydro-naphtalen-2-ylsulfanyl)-pent-2-en-4-ynoate d'éthyle

### (a) 6-(2,2-dichloro-vinylsulfanyl)-1,1,4,4,7-pentaméthyl-1,2,3,4-tetrahydro-naphtalène

De manière analogue à l'exemple 5(a), par réaction de 3,5,5,8,8-pentaméthyl-5,6,7,8-tetrahydro-naphtalène-2-thiol (15 g, 64,1 mmol) dans 500 ml de THF avec de l'hydrure de potassium 35% (11 g, 96,1 mmol), et du trichloroéthylène (6,91 ml, 76,9 mmol), on obtient 16,1 g (87%) du dérivé dichloré attendu sous forme d'huile incolore.
RMN1H (CDCl₃): 1,27 (12H, s), 1,67 (4H, s), 2,36 (3H, s), 6,46 (1H, s), 7,15 (1H, s), 7,38 (1H, s).

### (b) 6-éthynylsulfanyl-1,1,4,4,7-pentaméthyl-1,2,3,4-tetrahydro-naphtalène

De manière analogue à l'exemple 5(b), par réaction à -78°C de 6-(2,2-dichloro-vinylsulfanyl)-1,1,4,4,7-pentaméthyl-1,2,3,4-tetrahydro-naphtalène (8 g, 24,3 mmol) dans 200 ml de THF avec du nButyllithium 2,5 M / Hexane (21,4 ml, 53,5 mmol), on obtient 2,08 g (33%) de l'alcyne attendu sous forme d'un solide jaune.
RMN 1H (CDCl₃): 1,27 (12H,s), 1,67 (4H, s), 2,30 (3H, s), 3,15 (1H, s), 7,09 (1H, s), 7,58 (1H, s).

### (c) 3-méthyl-5-(3,5,5,8,8-pentaméthyl-5,6,7,8-tetrahydro-naphtalen-2-ylsulfanyl)-pent-2-en-4-ynoate d'éthyle

De manière analogue à l'exemple 5(c), par réaction de 6-ethynylsulfanyl-1,1,4,4,7-pentaméthyl-1,2,3,4-tetrahydro-naphtalene (2,08 g, 151 mmol), dans 60 ml de toluène avec Pd(OAc)2 (70 mg, 0,3 mmol), et TDMPP (140 mg), et Ethyl-2-butynoate (2,05 g, 20,9 mmol), on obtient 1 g (27%) de l'ester attendu sous forme d'huile brune.
RMN1H (CDCl3) : 1,29(12H, m), 1,67 (4H, s), 2,30 (3H, s), 2,37 (3H, s), 4,17 (2H, q), 6,01 (1H, d), 7,10 (1H, s), 7,53 (1H, s).

### EXEMPLE 8 :

### Acide 3-méthyl-5-(3,5,5,8,8-pentaméthyl-5,6,7,8-tetrahydro-naphtalen-2-vlsulfanyl)-pent-2-en-4-ynoique

De la soude (400 mg, 10 mmol), est additionnée à la solution du 3-méthyl-5-(3,5,5,8,8-pentaméthyl-5,6,7,8-tetrahydro-naphtalen-2-ylsulfanyl)-pent-2-en-4-ynoate d'éthyle (400 mg, 1,6 mmol), dans 15 ml de THF. Le milieu est chauffé 4 h à reflux. Il est ensuite versé sur un mélange éther éthylique /eau, acidifié à pH 1 par une solution d'acide chlorhydrique concentré et extrait à l'éther éthylique. Après décantation, la phase organique est lavée 2 fois à l'eau, séchée sur sulfate de magnésium anhydre et concentrée à l'évaporateur rotatif sous vide à 40°C. Le résidu est ensuite trituré dans l'heptane puis filtré.
Solide marron. Masse 240 mg. Rendement 44%. Tf : 165°C.
RMN1H (CDCl₃) : 2,31 (3H, s), 2,38 (3H, d), 6,02 (1H, d), 7,10 (1H, s), 7,53 (1H, s).

### EXEMPLE 9

### 3-Methyl-5-(5,5,8,8-tetramethyl-3-propoxy-5,6,7,8-tetrahydro-naphthalen-2-ylselanyl)-pent-2-en-4-ynoate d'éthyle.

### a) 5,5,8,8-tetramethyl-3-propoxy-5,6,7,8-tetrahydro-naphthalen-2-yl disélenide

De manière analogue à l'exemple 1(a), à partir de 6 g de 2-bromo-5,5,8,8-tetramethyl-3-propoxy-5,6,7,8-tetrahydro-naphthalen, on obtient 3,2 g (55%) du composé attendu sous forme d'une poudre orangée. Tf= 94°C.

### b) 3-Methyl-5-(5,5,8,8-tetramethyl-3-propoxy-5,6,7,8-tetrahydro-naphthalen-2-ylselanyl)-pent-2-en-4-ynoate d'éthyle.

De manière analogue à l'exemple 1(c), à partir de 380 mg de 3-méthyl-pent-2-en-4-ynoate d'éthyle et de 1 g du produit précédemment obtenu, on obtient 740 mg (58%) du composé attendu sous forme d'une poudre beige. Tf= 58°C.
RMN1H/CDCl3:1.05(t,3H); 1.27à1.32(m;12H); 1.68(s,4H); 1.80(sext,2H); 2.41(d,3H); 3.97(t,2H); 6.06(d,1H); 6.72(s,1H); 7.26(s,1H); 7.54(s,1H)
RMN 13C/CDCl3:11.0;CH3/ 14.6;CH3/ 20.1;CH3/ 22.9;CH2/ 32.3;4*CH3/34.5;Cq/ 34.9;Cq/ 35.4;2*CH2/ 60.4;CH2/ 70.6;CH2/ 77.6;Cq/ 107.3;Cq/109.5;CH/ 115.3;Cq/ 122.6;CH/ 126.8;CH/ 137.7;Cq/ 139.2;Cq/ 145.6;Cq/153.6;Cq/ 166.6;Cq

### EXEMPLE 10

### Acide 3-Methyl-5-(5,5,8,8-tetramethyl-3-propoxy-5,6,7,8-tetrahydro-naphthalen-2-ylselanyl)-pent-2-en-4-ynoïque.

De manière analogue à l'exemple 2, à partir de 570 mg du produit de l'exemple 9, on obtient 390 mg (73%) du composé attendu sous forme d'une poudre beige. Tf= 170°C.
RMN 1H/CDCl3: 1.05(t,3H); 1.28(m,12H); 1.67(s,4H); 2.39(d,3H); 3.97(t,2H); 6.07(d,1H); 6.72(s,1H); 7.54(s,1H)
RMN13C/CDCl3: 11.0;CH3/ 20.1;CH3/ 22.9;CH2/ 32.2;2*CH3/ 32.4;2*CH3/34.5;Cq/ 34.9;Cq/ 35.4;2*CH2/ 70.6;CH2/ 107.4;Cq/ 109.5;CH/ 115.3;Cq/123.3;CH/ 126.8;CH/137.5;Cq/ 139.3;Cq/ 145.6;Cq/ 153.5;Cq/ 168.5;Cq

### EXEMPLE 11

### 3-Methyl-5-(5,5,8,8-tetramethyl-4-propoxy-5,6,7,8-tetrahydro-naphthalen-2-ylselanyl)-pent-2-en-4-ynoate d'éthyle.

### a) 5,5,8,8-tetramethyl-4-propoxy-5,6,7,8-tetrahydro-naphthalen-2-yl disélenide

De manière analogue à l'exemple 1(a), à partir de 3,8 g de 2-bromo-5,5,8,8-tetramethyl-4-propoxy-5,6,7,8-tetrahydro-naphthalen, on obtient 1,8 g (47%) du composé attendu sous forme d'une poudre orangée. Tf= 75°C.

### b) 3-Methyl-5-(5,5,8,8-tetramethyl-4-propoxy-5,6,7,8-tetrahydro-naphthalen-2-ylselanyl)-pent-2-en-4-ynoate d'éthyle.

De manière analogue à l'exemple 1(c), à partir de 380 mg de 3-méthyl-pent-2-en-4-ynoate d'éthyle et de 1,1 g du produit précédemment obtenu, on obtient 900 mg (70%) du composé attendu sous forme d'une huile jaune.
RMN 1H/CDCl3:1.08't,3H); 1,27(m,6H); 1.39(s,6H); 1.63(m,4H); 1.87(sext,2H); 2.35(d,3H); 3.90(t,2H); 4.16(q,2H); 6.01(d,1H); 6.85(d,1H); 7.07(d,1H)
RMN13C/CDCl3: 10.7;CH3/ 13.9;CH3/ 19.2;CH3/ 22.4;CH2/ 28.0;2*CH3/31.6;2*CH3/ 34.0;Cq/ 34.5;CH2/ 34.7;Cq/ 37.6;CH2/ 59.7;CH2/ 69.5;CH2/77.3;Cq/ 105.2;Cq/ 109.6;CH/ 119.8;CH/ 122.0;CH/ 132,3;Cq/ 136.9;Cq/148.4;Cq/ 158.5;Cq/ 165.8;Cq

### EXEMPLE 12

### Acide 3-Methyl-5-(5,5,8,8-tetramethyl-4-propoxy-5,6,7,8-tetrahydro-naphthalen-2-ylselanyl)-pent-2-en-4-ynoïque.

De manière analogue à l'exemple 2, à partir de 750 mg du produit de l'exemple 11, on obtient 430 mg (61%) du composé attendu sous forme d'une poudre beige. Tf= 164°C.
RMN 1H/CDCl3: 1.09(t,3H); 1.27(s,6H); 1.38(s,6H); 1.63(m,4H); 1.88(sext,2H); 2.36(d,3H); 3.93(t,2H); 6.02(d,1H); 6.84(d,1H); 7.08(d,1H)
RMN13C/CDCl3: 11.3;CH3/ 20.2;CH3/ 22.9;CH2/ 28.6;2*CH3/ 32.1;2*CH3/34.5;Cq/ 35.0;Cq/ 35.3;CH2/ 38.1;CH2/ 70.0;CH2/ 80.4;Cq/ 105.7;Cq/110.2;CH/ 120.5;CH/ 121.4;CH/ 133.0;Cq/ 140.4;Cq/ 149.05;Cq/ 159.1;Cq/171.3;Cq

### EXEMPLE 13

### 5-(4-Methoxymethoxy-5,5,8,8-tetramethyl-5,6,7,8-tetrahydro-naphthalen-2-ylselanyl)-3-methyl-pent-2-en-4-ynoate d'éthyle.

### a) 4-methoxymethoxy-5,6,7,8-tetrahydro-5,5,8,8-tetramethylnaphtalene-2-disélénide.

De manière analogue à l'exemple 1(a), à partir de 4 g de 2-bromo-5,5,8,8-tetramethyl-4-methoxymethoxy-5,6,7,8-tetrahydro-naphthalen, on obtient 2,4 g (61%) du composé attendu sous forme d'une poudre orangée. Tf= 91°C.

### b) 5-(4-Methoxymethoxy-5,5,8,8-tetramethyl-5,6,7,8-tetrahydro-naphthalen-2-ylselanyl)-3-methyl-pent-2-en-4-ynoate d'éthyle.

De manière analogue à l'exemple 1(c), à partir de 400 mg de 3-méthyl-pent-2-en-4-ynoate d'éthyle et de 800 mg du produit précédemment obtenu, on obtient 648 mg (96%) du composé attendu sous forme d'un solide brun. Tf= 50°C.
RMN 1H/CDCl3: 1.25à1.28(m,9H); 1.39(s,6H); 1.63(m,4H); 2.35(d,3H); 3.50(3H); 4.16(q,2H); 5.21(s,2H); 6.06(d,1H); 7.12(d,1H); 7.17(d,1H)
RMN13C/CDCl3: 14.5;CH3/ 19.7;CH3/ 28.7;2*CH3/ 32.1;2*CH3/ 34.6;Cq/35.0;CH2/ 35.3;Cq/ 38.0;CH2/ 56.3;CH3/ 60.2;CH2/ 77.7;Cq/ 94.6;CH2/106.1;Cq/ 112.6;CH/ 121.2;CH/ 122.6;CH/ 125.5;Cq/ 133.1;Cq/ 137.5;Cq/149.0;Cq/ 157.6;Cq/ 166.4;Cq

### EXEMPLE 14

### Acide 5-(4-Methoxymethoxy-5,5,8,8-tetramethyl-5,6,7,8-tetrahydro-naphthalen-2-ylselanyl)-3-methyl-pent-2-en-4-ynoïque.

De manière analogue à l'exemple 2, à partir de 440 mg du produit de l'exemple 13, on obtient 330 mg (80%) du composé attendu sous forme d'une poudre beige. Tf= 130°C.
RMN 1H/CDCl3: 1.27(s,6H); 1.38(s,6H); 1.64(m,4H); 2.36(d,3H); 3.50(s,3H); 5.21 (s,2H); 6.09(d,1 H); 7.11(d,1H); 7.18(d,1H)
RMN13C/CDCl3: 19.9;CH3/ 28.6;2*CH3/ 32.0;2*CH3/ 34.5;CH2/ 34.8;Cq/35.2;Cq/ 37.9;CH2/ 56.2;CH3/ 79.8;Cq/ 94.5;CH2/ 106.2;Cq/ 112.5;CH/121.2;CH2/ 121.4;CH2/ 125.3;Cq/ 133.2;Cq/ 140.3;Cq/ 149.0;Cq/ 157.6;Cq/171.4;Cq

### EXEMPLE 15

### 5-(3-Hydroxy-5,5,8,8-tetramethyl-5,6,7,8-tetrahydro-naphthalen-2-ylselanyl)-3-methyl-pent-2-en-4-ynoate d'éthyle.

### a) 3-methoxymethoxy-5,6,7,8-tetrahydro-5,5,8,8-tetramethylnaphtalene-2-disélénide.

De manière analogue à l'exemple 1(a), à partir de 13,5 g de 2-bromo-5,5,8,8-tetramethyl-3-methoxymethoxy-5,6,7,8-tetrahydro-naphthalen, on obtient 5,85 g (43%) du composé attendu sous forme d'une poudre orangée. Tf= 95°C.

### b) 3-hydroxy-5,6,7,8-tetrahydro-5,5,8,8-tetramethylnaphtalene-2-disélénide

Un mélange du produit de l'exemple 15a (200 mg) d'acide sulfurique concentré (1,4 ml) de méthanol (20ml) et de THF (20 ml) est agité 6h à température ambiante. Le milieu réactionnel est extrait à l'acétate d'éthyle. La phase organique est lavée deux fois à l'eau séchée sur sulfate de magnésium et concentré à l'évaporateur rotatif sous vide. Le produit est purifié par chromatographie flash (CH₂Cl₂ :5/ Heptane :5). On obtient 120 mg (63%) du composé attendu sous forme d'une poudre orangée.
RMN 1H/CDCl3: 1.17(s,6H); 1.25(s,6H); 1.63(s,4H); 6.93(s,1H); 7.34(s,1H)

### c) 5-(3-Hydroxy-5,5,8,8-tetramethyl-5,6,7,8-tetrahydro-naphthalen-2-ylselanyl)-3-methyl-pent-2-en-4-ynoate d'éthyle.

De manière analogue à l'exemple 1(c), à partir de 980 mg de 3-méthyl-pent-2-en-4-ynoate d'éthyle et de 2 g du produit précédemment obtenu, on obtient 1,29 g (87%) du composé attendu sous forme d'un solide jaune. Tf= 50°C.
RMN 1H/CDCl3: 1.26à1.27(m,12H); 1.66(s,4H); 2.30(s,3H); 4.16(q,2H); 5.48(s,1 H); 6.00(s,1H); 6.88(s,1 H); 7.49(s,1H)
RMN 13C/CDCl3: 14.2;CH3/ 19.5;CH3/ 31.7;2*CH3/ 32.0;2*CH3/ 34.0;Cq/34.5;Cq/ 34.8;CH2 /35.0;CH2/ 60.1;CH2/ 77.2;Cq/ 103.4;Cq/ 110.8;Cq/113.4;CH/ 123.3;CH/ 131.2;CH/ 137.0;Cq/ 139.1;Cq/ 148.6;Cq/ 152.2;Cq/;Cq

### EXEMPLE 16

### Acide 5-(3,5,5,8,8-Pentamethyl-5,6,7,8-tetrahydro-naphthalen-2-ylselanyl)-3-phenyl-pent-2-en-4-ynoïque.

### a) 3-phényl-5-triméthylsilanyl-pent-2-en-4-ynoate d'éthyle.

Une solution de TDMPP (1,4g), de palladium acétate (700 mg) dans le THF (200ml) est agitée 15 mn à température ambiante puis, le phenyl propiolate de methyl (10g, 62,5 mmol) est additionné . Le milieu réactionnel est agité 5 mn à température ambiante puis, le trimethyl silyl acétylène (87,5 mmol) est additionné. L'agitation est poursuivie une nuit à température ambiante. Le milieu réactionnel est concentré et filtré sur silice.
RMN 1H/CDCl3: 0.28(s,9H); 3.67(s,3H); 6.45(s,1H); 7.39à7.41(m,3H); 7.48à7.51(m,2H)

### b) 3-phénylpent-2-en-4-ynoate d'éthyle.

De manière analogue à l'exemple 1(b), l'huile précédemment obtenue est traitée par 10 g de fluorure de potassium. On obtient 6,8 g (58%) du composé attendu sous forme d'une huile brune.
RMN 1H/CDCl3: 3.31(s,1H); 361(s,1H); 6.38(s,1H); 7.33à7.46(m,5H)
RMN 13C/CDCl3: 52.0;CH3/ 83.4;CH/ 87.1;Cq/ 126.3;CH/ 128.4;2*CH/129.6;2*CH/ 131.0;CH/ 136.1;Cq/ 137.5;Cq/ 165.9;Cq.

### c) Acide 3-phénylpent-2-en-4-ynoique.

De manière analogue à l'exemple 2, à partir de 5 g du produit précédent, on obtient 4,8 g (100%) du composé attendu sous forme d'une poudre orange. Tf= 130°C.
RMN 1H/DMSO: 4.53(s,1H); 6.0(s,1H); 7.38(s,5H)
RMN 13C/DMSO: 84.2;Cq/ 85.7;Cq/ 128.2;CH/ 128.4;CH/ 128.5;2*CH/129.2;CH/ 133.7;Cq/ 136.1;Cq

### d) Acide 5-(3,5,5,8,8-Pentamethyl-5,6,7,8-tetrahydro-naphthalen-2-ylselanyl)-3-phenyl-pent-2-en-4-ynoïque.

De manière analogue à l'exemple 1(c), à partir de 1 g du produit précédemment obtenu et de 1,95 g 5,6,7,8-tetrahydro-3,5,5,8,8-pentaméthylnaphtalene-2-disélénide, on obtient 1,1 g (70%) du composé attendu sous forme d'un solide brun. Tf= 170°C.
RMN 1H/CDCl3: 1.16(s,6H); 1.27(s,6H); 1.62(s,4H); 2.29(s,3H); 6.23(s,1H); 7.07(s,1H); 7.31à7.48(m,6H)
RMN 13C/CDCl3: 32.1;4*CH3/ 34.4;Cq/ 34.5;Cq/ 35.3;2*CH2/ 82.1;Cq/104.8;Cq/ 121.9;CH/ 125.6;Cq/ 128.5;CH/ 129.9;CH/ 129.0;3*CH/ 129,6;2*CH/134.3;Cq/ 136.2;Cq/ 141.3;Cq/ 144.8;Cq/ 145.3;Cq/ 170.7;Cq

### EXEMPLE 17

### Acide 5-(4-Methoxymethoxy-5,5,8,8-tetramethyl-5,6,7,8-tetrahydro-naphthalen-2-ylselanyl)-3-phenyl-pent-2-en-4-ynoïque.

De manière analogue à l'exemple 1(c), à partir de 2,12 g du produit obtenu dans l'exemple 16(c) (Acide 3-phénylpent-2-en-4-ynoique) et de 1,04 g de 4-methoxymethoxy-5,5,8,8-tetramethyl-5,6,7,8-tetrahydro-naphthalen-2-diselenide, on obtient 700 mg (97%) du composé attendu sous forme d'un solide brun. Tf= 122°C.
RMN 1H/DMSO: 1.13(s,6H); 1.33(s,6H); 1.55à1.60(m,4H); 3.39(s,3H); 5.13(s,2H); 6.33(s,1H); 7.06(s,1H); 7.14(s,1H); 7.35à7.45(m,5H)
RMN 13C/DMSO: 28.6;2*CH3/ 31.8;2*CH3/ 34.3;CH2/ 34.5;Cq/ 35.0;Cq/37.6;CH2/ 56.3;CH3/ 78.3;Cq/ 94.2;CH2/ 104.7;Cq/ 112.0;CH/ 120.6;CH/125.2;Cq/ 125.3;CH/ 128.5;4*CH/ 129.2;CH/ 132.3;Cq/ 135.2;Cq/ 136.1;Cq/148.8;Cq/ 157.0;Cq/ 166.3;Cq

### EXEMPLE 18

### 5-(4-Hydroxy-5,5,8,8-tetramethyl-5,6,7,8-tetrahydro-naphthalen-2-ylselanyl)-3-phenyl-pent-2-en-4-ynoate de méthyle.

De manière analogue à l'exemple 15(b), à partir de 300 mg du produit obtenu dans l'exemple 17, on obtient 60 mg (23%) du composé attendu sous forme d'un solide brun. Tf= 93°C.
RMN 1H/CDCl3: 1.8(s,6H); 1.39(s,6H); 1.57à1.66(m,4H);3.62(s,3H); 4.77(s,1H); 6.24(s,1H); 6.48(s,1H); 6.95(s,1 H); 7.38à7.49(m,5H)
RMN 13C/CDCl3: 28.1;2*CH3/ 31.9;2*CH3/ 34.0;Cq/34.8;CH2/ 34.9;Cq/37.5;CH2/ 51.5;CH3/ 80.2;Cq/ 104.9;Cq/ 113.9;CH/ 121.8;CH/ 124.8;Cq/128.0;2*CH/ 128.7;2*CH/ 129.0;CH/ 130.1;Cq/ 136.4;Cq/ 138.6;Cq/ 149.5;Cq/165.7;Cq

### EXEMPLE 19

### Acide 3-propyl-5-(5,5,8,8-tetraméthyl-5,6,7,8,-tetrahydro-naphtalen-2-ylselanyl)-pent-2-en-4-ynoique.

### (a) 5-trimethylsilyl-3-propyl-pent-2-en-4-ynoate d'éthyle.

De manière analogue à l'exemple 16(a), à partir de 95,2 mmol de trimethylsilyl acétylène et de 10 g d'hexynoate de méthyle, on obtient le composé attendu sous forme d'une huile brune.
RMN 1H/CDCl3: 0.01 (s,9H); 0,75(t,3H); 1.40(q,2H); 2.50(t,2H); 3.49(s,3H); 5.88(s,1H)
RMN 13C/CDCl3: 16.0;CH3/ 17.7;CH2/ 36.5;CH2/ 53.6;CH3/ 68.3;CH/ 86.6;Cq/127.8;CH/ 144.4;Cq/ 168.3;Cq

### b) 3-propyl-pent-2-en-4-ynoate d'éthyle.

De manière analogue à l'exemple 16(b), à partir de l'huile précédemment obtenue et de 10g de fluorure de potassium, on obtient 2,5 g (15%) du composé attendu sous forme d'une huile brune.
RMN 1H/CDCl3: 0.77(t,3H); 1.43(q,2H); 2.54(t,2H); 3.01(s,1H); 3.52(s,3H); 5.95(s,1H)
RMN 13C/CDCl3: 15.4;CH3/ 23.4;CH2/ 35.8;CH2/ 53.0;CH3/ 83.8;CH/ 86.2;Cq/127.1;CH/ 143.7;Cq/ 167.7;Cq

### c) Acide 3-propyl-pent-2-en-4-ynoïque.

De manière analogue à l'exemple 16(c), à partir de l'huile précédemment obtenue, on obtient 1,86 (82%) du composé attendu sous forme d'un solide brun.
RMN 1H/CDCl3: 0.99(t,3H); 1.65(quint,2H); 2.74(t,2H); 3.275s,1H); 6.16(s,1H)
RMN 13C/CDCl3; 15.4;CH3/ 23.3;CH2/ 35.8;CH2/ 84.9;Cq/ 85.7;CH/ 126.7;CH/146.3;Cq/ 172.6;Cq

### d) Acide 3-propyl-5-(5,5,8,8-tetraméthyl-5,6,7,8,-tetrahydro-naphtalen-2-ylselanyl)-pent-2-en-4-ynoique.

De manière analogue à l'exemple 16(d), à partir de 600 mg du produit précédemment obtenu et de 1,2g de 5,6,7,8-tetrahydro-3,5,5,8,8-pentaméthylnaphtalene-2-disélénide (exemple 3a), on obtient 800 mg (45%) du composé attendu sous forme d'un solide blanc. Tf=127°C.
RMN 1H/CDCl3: 0.85(t,3H); 1.17(d,12H); 1.56(t,6H); 2.2(s,3H); 2.69(t,2H); 5.94(s,1H); 6.99(s,1H); 7.50(,1H)

### EXEMPLE 20

### Acide 5-(4-Benzyloxy-3-bromo-5,5,8,8-tetramethyl-5,6,7,8-tetrahydro-naphthalen-2-ylselanyl)-3-methyl-pent-2-en-4-ynoïque.

### a) 4-hydroxy-5,6,7,8-tetrahydro-5,5,8,8-tetramethylnaphtalene-2-disélénide

Le produit 13(a), 4-methoxymethoxy-5,6,7,8-tetrahydro-5,5,8,8-tetramethylnaphtalene-2-disélénide (12,4g) est traité de manière analogue à l'exemple 15(b), on obtient 11 g (100%) du composé attendu sous forme d'un solide blanc. Tf=200°C.
RMN 1H/CDCl3: 1.22(s,6H); 1.42(s,6H); 1.63(m,4H); 5.25(s,1H); 6.75(d,1H); 7.11(d,1H)
RMN 13C/CDCl3: 28.5;2*CH3/ 32.2; 2*CH3/ 34.4;2*Cq/ 35.3;CH2/ 38.0;CH2/116.6;CH/ 122.6;CH/ 128.1;Cq/ 131.5;Cq/ 149.1;Cq/ 155.5;Cq

### b) 4-benzyloxy-5,6,7,8-tetrahydro-5,5,8,8-tetramethylnaphtalene-2-disélénide

Un mélange du produit précédemment obtenu (2,5 g, 4,4 mmol), de carbonate de césium (2,95 g) et de chlorure de benzyle (1,3 ml) dans le DMF (18 ml) est agité à température ambiante pendant 24 h. Le milieu réactionnel est extrait par de l'éther éthylique. La phase organique est lavée à l'eau, séchée sur sulfate de magnésium et concentrée à l'évaporateur rotatif. Le produit est purifié par filtration sur silice (heptane puis dichlorométhane). On obtient 2,1 g (63%) du composé attendu sous forme d'une poudre jaune.
RMN 1H/CDCl3: 1,21(s,6H); 1. 34(s,6H); 1.59 (m,4H); 4.96 (s,2H); 7.02 (d,1H); 7.21(d,1H); 7.29 à 7.41 (m, 5H).

### c) Acide 3-methyl-pent-2-en-4-ynoïque.

De manière analogue à l'exemple 16(c), à partir du produit de l'exemple 1(b), on obtient le composé attendu sous forme d'un solide brun.

### d) Acide 5-(4-Benzyloxy-3-bromo-5,5,8,8-tetramethyl-5,6,7,8-tetrahydro-naphthalen-2-ylselanyl)-3-methyl-pent-2-en-4-ynoïque.

De manière analogue à l'exemple 1(c), à partir de 430 mg du produit précédemment obtenu et de 1g de 4-benzyloxy-5,6,7,8-tetrahydro-5,5,8,8-tetramethylnaphtalene-2-disélénide, on obtient 100 mg (11%) du composé attendu sous forme d'un solide brun.
RMN 1H/CDCl3: 1.1(s,6H); 1.26(s,6H); 1.35(m,4H); 2.05(s,3H); 4.83(s,2H); 5.76(s,1H); 6.96(s,1H); 7.02à7.24(m,6H)

### EXEMPLE 21

### Acide 5-(4-Benzyloxy-5,5,8,8-tetramethyl-5,6,7,8-tetrahydro-naphthalen-2-ylselanyl)-3-phenyl-pent-2-en-4-ynoïque.

De manière analogue à l'exemple 1(c), à partir de 671 mg d'acide 3-phénylpent-2-en-4-ynoique (exemple 16(c)) et de 1g de 4-benzyloxy-5,6,7,8-tetrahydro-5,5,8,8-tetramethylnaphtalene-2-disélénide, on obtient 240 mg (23%) du composé attendu sous forme d'un solide blanc.
RMN 1H/THF: 1.44(s,6H); 1.66(s,6H); 1.72(m,4H); 4.74(s,2H); 6.47(s,1H); 7.08à7.62(m,12H)
RMN 13C/THF: 31.5;2*CH3/ 34.4;2*CH3/ 37.4;Cq/ 37.9;Cq/ 38.2;CH2/39.5;CH/ 70.7;CH2/ 79.7;Cq/ 107.2;Cq/ 111.0;CH/ 113.5;Cq /128.0à129.1;7*CH/ 133.3;Cq/ 136.4;Cq/ 136.5;Cq/ 136.6;Cq/ 136.8;Cq/146.4;Cq/ 158.5;Cq

### EXEMPLE 22

### 5-[5-Adamantan-1-yl-4-(2-methoxy-ethoxymethoxy)-2-methyl-phenylselanyl]-3-methyl-pent-2-en-4-ynoate d'éthyle.

### a) 5-Adamantan-1-yl-4-(2-methoxy-ethoxymethoxy)-2-methyl-phenyl disélénide.

Une petite portion d'une solution de 2-(adamantan-1-yl)-4-bromo-5-methyl-1-méthoxyéthoxyméthoxyphényl (17 g, 41,5 mmol) dans le THF (160 ml) est coulée sur un mélange de magnésium (1,51 g) et un cristal d'iode, en chauffant légèrement. Quand le milieu réactionnel se décolore le reste de la solution est additionnée de manière à maintenir un léger reflux. Après la fin de l'addition, la solution est chauffée à reflux 1 h. Après retour à température ambiante, 3,6 g de sélénium sont additionnés. Le milieu réactionnel est agité 3 h. à température ambiante, puis une solution d'acide chlorhydrique 1N (105 ml) et de l'éther éthylique sont additionnés au milieu réactionnel. La phase organique est lavée à l'eau, séchée sur sulfate de magnésium et concentrée à l'évaporateur rotatif. Puis, on additionne de l'hydroxyde de sodium (131 mg) et de l'éthanol (27 ml). La suspension est agitée à l'air et à température ambiante 12 h. Le produit est isolé par filtration puis, la soude est éliminée par filtration sur silice le dichlorométhane. On obtient 12 g (71%) d'un solide jaune. Tf=101°C.
RMN 1H/CDCl3: 1.73 (s,6H); 2.00 (s,9H); 2.30 (s,3H); 3.40 (s,3H); 3.59 (m,2H); 3.83 (m,2H); 5.29 (s,2H); 6.95 (s,1H); 7.48 (s,1H).

### b) 5-[2-(adamantan-1-yl)-5-methyl-1-méthoxyéthoxyméthoxyphen-4-ylselanyl)-3-methyl-pent-2-en-4-ynoate d'éthyle.

Le produit est obtenu de manière analogue à l'exemple 1(c). Cependant, l'addition de brome se fait à 0°C et le mélange brome, disélénide est agité seulement 5 mn avant l'addition de l'iodure de cuivre, du DMF et de l'alcyne. A partir de 150 mg de 3-Méthyl pent-2-en-4-ynoate d'éthyle (exemple 1(b)) et de 500 mg du produit précédemment obtenu, on obtient le composé attendu sous forme d'une huile incolore.
RMN 1H/CDCl3: 1.27 (s, 3H) ; 1.77 (s,6H); 2.09 (s,9H); 2.33 (s,3H); 3.41 (s,3H); 3.59 (m,2H); 3.84 (m,2H); 4.16 (q, 2H) ; 5.30 (s,2H); 6.03 (s, 1H) ; 7.02 (s,1H); 7.56 (s,1H).

### EXEMPLE 23

### Acide 5-[5-Adamantan-1-yl-4-(2-methoxy-ethoxymethoxy)-2-methyl-phenylselanyl]-3-methyl-pent-2-en-4-ynoïque.

De manière analogue à l'exemple 2, à partir du produit de l'exemple 22, on obtient le composé attendu sous forme d'un solide blanc.
RMN 1H/CDCl3: 1.76 (s,6H); 2.09 (s,9H); 2.34 (s,3H); 3.40 (s,3H); 3.60 (m,2H); 3.84 (m,2H); 5.30 (s,2H); 6.03 (s, 1H) ; 7.03 (s,1H); 7.55 (s,1H).

### EXEMPLE 24

### Acide 5-[5-Adamantan-1-yl-4-(2-methoxy-ethoxymethoxy)-2-methyl-phenylselanyl]-3-propyl-pent-2-en-4-ynoïque.

Le produit est obtenu de manière analogue à l'exemple 22(b). A partir de 150 mg de 3-propyl pent-2-en-4-ynoic acide (exemple 19(c)) et de 500 mg de 2-methyl-4-méthoxyéthoxyméthoxy-5-(adamantan-1-yl)-1-disélénide, on obtient le composé attendu sous forme d'un solide jaunâtre.
RMN 1H/CDCl3: 0.90 (t, 3H) ; 1.64 (q, 2H) ; 1.27 (s, 3H) ; 1.77 (s,6H); 2.09 (s,9H); 2.34 (s, 3H); 2.78 (t, 2H), 3.41 (s,3H); 3.60 (m,2H); 3.85 (m,2H); 5.30 (s,2H); 6.03 (s, 1H) ; 7.03 (s,1H); 7.54 (s,1H).

### EXEMPLE 25

### Acide 5-[5-Adamantan-1-yl-4-(2-methoxy-ethoxymethoxy)-2-methyl-phenylselanyl]-3-phenyl-pent-2-en-4-ynoïque.

Le produit est obtenu de manière analogue à l'exemple 22(b). A partir de 168 mg de 3-phenyl-pent-2-en-4-ynoic acid (exemple 16(c)) et de 500 mg de 2-methyl-4-méthoxyéthoxyméthoxy-5-(adamantan-1-yl)-1-disélénide, on obtient le composé attendu sous forme d'un solide brun.
RMN 1H/CDCl3: 1.71 (s,6H); 1.98 (s,9H); 2.31 (s, 3H); 3.40 (s,3H); 3.59 (m,2H); 3.83 (m,2H); 5.29 (s,2H); 6.22 (s, 1H) ; 7.00 (s, 1H); 7.29 à 7.46 (m,6H).

### EXEMPLE 26

### 5-(3,5-Di-tert-butyl-2-methoxymethoxy-phenylselanyl)-3-methyl-pent-2-en-4-ynoate d'éthyle.

### a) 2-bromo-4,6-di-tert-butyl-1-méthoxyméthoxyphenyl.

De manière analogue à l'exemple 20 (b), à partir de 2-bromo-4,6-di-*tert*-butyl-phenol, de césium carbonate et de chlorure de methoxymethyle, on obtient le produit sous forme d'une huile.

### b) 4,6-di-tert-butyl-1-méthoxyméthoxyphen-2-yl diselenide.

De manière analogue à l'exemple 22 (a), à partir de 10 g du produit obtenu précédemment, de 1,1 g de magnésium et de 2,63 g de sélénium, on obtient 7,6 g (76%) du produit attendu sous forme d'un solide jaune.
RMN 1H/CDCl3: 1.18 (s, 9H); 1.42 (s, 9H); 3.68 (s, 3H); 5.08 (s,2H); 7.23 (d,1H); 7.54 (d, 1H).

### c) 5-(3,5-Di-tert-butyl-2-methoxymethoxy-phenylselanyl)-3-methyl-pent-2-en-4-ynoate d'éthyle.

De manière analogue à l'exemple 22(b), à partir de 617 mg du produit obtenu précédemment, de 232 mg de 3-méthyl-pent-2-en-4-ynoate d'éthyle, on obtient le produit attendu sous forme d'une huile incolore.
RMN 1H/CDCl3: 1.28 (t, 3H) ; 1.32 (s, 9H); 1.40 (s, 9H); 2.37 (d, 3H); 3.67 (s, 3H) ; 4.18 (q, 2H) ; 5.01 (s,2H); 7.29 (d,1H); 7.63 (d, 1H).

### EXEMPLE 27

### Acide 5-(3,5-Di-tert-butyl-2-methoxymethoxy-phenylselanyl)-3-methyl-pent-2-en-4-ynoïque.

De manière analogue à l'exemple 2, à partir du produit de l'exemple 26, on obtient le composé attendu sous forme d'un solide blanc.
RMN 1H/CDCl3: 1.33 (s, 9H); 1.40 (s, 9H); 2.38 (d, 3H); 3.67 (s, 3H) ; 5.01 (s,2H); 6.05 (d, 1H) ; 7.29 (d,1H); 7.63 (d, 1H).

### EXEMPLE 28

### 3-Methyl-5-(5,5,8,8-tetramethyl-3-hexyloxy-5,6,7,8-tetrahydro-naphthalen-2-ylselanyl)-pent-2-en-4-ynoate d'éthyle.

### a) 4-hexyloxy-5,6,7,8-tetrahydro-5,5,8,8-tetramethylnaphtalene-2-disélénide

De l'hydrure de sodium à 60% (225 mg, 5,63 mmol) est additionné par fractions à une solution de 4-hydroxy-5,6,7,8-tetrahydro-5,5,8,8-tetramethylnaphtalene-2-disélénide (1,2 g, 2,56 mmol) dans 15 ml de THF et 15 ml de THF. L'agitation est poursuivie 30 mn à température ambiante après la fin de l'addition, puis du iodohexane (1 ml, 6,8 mmol) est additionné. Le milieu réactionnel est agité 4 h. à T.A. puis est traité avec de l'eau et de l'éther éthylique. La phase organique est lavée à l'eau, séchée sur sulfate de magnésium et concentrée. Après purification par fast plug (heptane 95, CH2Cl2 5), le produit est obtenu sous forme d'une huile jaune.
RMN 1H/CDCl3: 0.90 (m, 9H) ; 1.30 à 1.48 (m,12H); 1.59 (m,4H); 1.77 (m, 2H) ; 3.85 (t, 2H), 6.92(d,1H); 7.17(d,1H).

### b) 3-Methyl-5-(5,5,8,8-tetramethyl-3-hexyloxy-5,6,7,8-tetrahydro-naphthalen-2-ylselanyl)-pent-2-en-4-ynoate d'éthyle.

De manière analogue à l'exemple 22(b), à partir de 689 mg du produit obtenu précédemment, de 232 mg de 3-méthyl-pent-2-en-4-ynoate d'éthyle, on obtient le produit attendu sous forme d'une huile incolore.
RMN 1H/CDCl3: 0.90 (m, 9H) ; 1.26 à 1.38 (m,15H); 1.56 (m,4H); 1.85 (m, 2H) ; 2.35 (s, 3H); 3.95 (t, 2H), 4.17 (q, 2H) ; 6, 00 (d, 1H); 6.84(d,1H); 7.07(d,1H).

### EXEMPLE 29

### Acide 3-Methyl-5-(5,5,8,8-tetramethyl-3-hexyloxy-5,6,7,8-tetrahydro-naphthalen-2-ylselanyl)-pent-2-en-4-ynoïque.

De manière analogue à l'exemple 2, à partir de 513 mg du produit de l'exemple 27, on obtient le composé attendu sous forme d'un solide blanc.
RMN 1H/CDCl3: 0.90 (m, 9H) ; 1.30 (s, 12H) ; 1.38 (s,12H); 1.59 (m,4H); 1.85 (m, 2H) ; 2.35 (s, 3H) ; 3.95 (t, 2H), 6, 02 (s, 1H) ; 6.83 (d,1H); 7.08 (d,1H).

### EXEMPLE 30

### Acide 5-[4-Adamantan-1-yl-3-(2-methoxy-ethoxymethoxy)-phenylselanyl]-3-propyl-pent-2-en-4-ynoïque.

### a) 2-(adamantan-1-yl)-5-bromo-1-(2-methoxy-ethoxymethoxy)-phenyl

De manière analogue à l'exemple 28 (a), à partir de 20,9 g de 2-(adamantan-1-yl)-5-bromo-1-phenol, de 2,5 g d'hydrure de sodium et de 8,92 g de chlorure de methoxyethoxymethyle, on obtient 17 g (64%) de produit attendu sous forme d'un solide blanc. Tf=88°C.

### b) 4-Adamantan-1-yl-3-(2-methoxy-ethoxymethoxy)-phenyldisélénide.

De manière analogue à l'exemple 1 (a), à partir de 13,04 g de 2-(adamantan-1-yl)-5-bromo-1-méthoxyéthoxyméthoxyphenyl, on obtient 9,9 g (76%) de produit attendu sous forme d'une huile jaune.
RMN 1H/CDCl3: 1.55 (s, 6H) ; 2.05 (d,9H); 3.38 (s,3H); 3.57 (m, 2H) ; 3.82 (m, 2H) ; 5.27 (s, 2H), 7,11 (d, 1H) ; 7.22 (dd, 1H) ; 7.38 (d, 1H).

### c) Acide 5-[4-Adamantan-1-yl-3-(2-methoxy-ethoxymethoxy)-phenylselanyl]-3-propyl-pent-2-en-4-ynoïque.

De manière analogue à l'exemple 22(b), à partir de 600 mg du produit précédemment obtenu et de 202 mg de 3-propyl-pent-2-en-4-ynoic acid (exemple 19 (c)) , on obtient le produit attendu sous forme d'un solide blanc. Tf=95 °C.
RMN 1H/CDCl3: 0.97(t,3H); 1.66(q,2H); 1.76(s,6H); 2.07(s,9H); 2.80(t,2H); 3.38(s,3H); 3.61(m,2H); 3.85(m,2H); 5.33(s,2H); 6.12(s,1H); 7.08à7.35(m,3H)
RMN 13C/CDCl3: 13.7;CH3/ 21.9;CH2/ 29.0;CH/ 34.0;CH2/ 37.0;CH2/40.6;CH2/ 59.0;CH3/ 67.8;CH2/ 71.6;CH2/ 79.1;Cq/ 93.4;CH2/ 107;Cq/115.2;CH/ 122.2;CH/ 125.8;2*Cq/ 127.9;CH/ 13.3;2*Cq/ 157.3;Cq

### EXEMPLE 31

### 5-[2-(adamantan-1-yl)-1-méthoxyéthoxyméthoxyphen-5-ylselanyl)-3-methyl-pent-2-en-4-ynoate d'éthyle.

De manière analogue à l'exemple 22(b), à partir de 600 mg du produit de l'exemple 30 (b) et de 202 mg de 3-methyl-pent-2-en-4-ynoate d'éthyle (exemple 1 (b)), on obtient 300 mg (74%) du produit attendu sous forme d'une huile brune.
RMN 1H/CDCl3: 1.28(t,3H); 1.76(s,6H); 2.05(m,9H); 2.37(s,3H); 3.39(s,3H); 3.60(m,2H); 3.85(m,2H); 4.17(q,2H); 5.30(s,3H); 6.04(d, 1H); 7.09à7.32(m,3H)

### EXEMPLE 32

### 5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoate d'éthyle.

### a) 5-(2-methoxyethoxymethoxy)-7-thiol-1,1,4,4-tetraméthyl-1,2,3,4-tetrahydro-naphtalène

Une solution de tert-butyllithium 1,7 M dans le pentane (95 mmol, 56 ml) est additionnée à une solution de 7-bromo-5-(2-methoxyethoxymethoxy)-1,1,4,4-tetraméthyl-1,2,3,4-tetrahydro-naphtalène (16,02 g, 43 mmol) dans le THF (300 ml) à -78°C en 10 min. Le mélange est agité à 0°C 30 min. Le soufre (1,49 g, 46 mmol) est additionné en 2 fois à -70°C. Le mélange est agité 2,5 h. Une solution d'HCl 2N est additionnée, puis le mélange réactionnel est traité par de l'éther éthylique. La phase organique est lavée 2 fois à l'eau, séchée sur sulfate de magnésium anhydre et concentrée à l'évaporateur rotatif sous vide à 40°C. Le produit est purifié par chromatographie flash (heptane 9, AcOEt 1). Huile jaune. Masse : 3,73 g. Rendement : 29%.

### b) 7-(2,2-dichloro-vinylsulfanyl)-5-(2-methoxyethoxymethoxy)-1,1,4,4-tetraméthyl-1,2,3,4-tetrahydro-naphtalène.

De manière analogue à l'exemple 5 (a), à partir de 1,3 g de 5-(2-methoxyethoxymethoxy)-7-thiol-1,1,4,4-tetraméthyl-1 ,2,3,4-tetrahydro-naphtalène, de 688 mg d'hydrure de potassium à 35% et de 431 ul de trichloroethylène, on obtient 1,27 g (76%) du produit attendu sous forme d'une huile jaune.

### c) 7-Ethynylsulfanyl-5-(2-methoxy-ethoxymethoxy)-1,1,4,4-tetramethyl-1,2,3,4-tetrahydro-naphthalene

De manière analogue à l'exemple 5 (b), à partir de 625 mg du produit précédemment obtenu et de 1,3 ml d'une solution de n butyllithium (2,5M/Hexane), on obtient 410 mg (67%) du produit attendu sous forme d'une huile jaune.

### d) 5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoate d'éthyle.

De manière analogue à l'exemple 5 (c), à partir de 613 mg du produit précédemment obtenu et de 471 mg de butynoate d'éthyle, on obtient 162 mg (20%) de l'isomère E attendu sous forme d'une huile jaune et 59 mg (7%) de l'isomère Z sous forme d'une huile jaune.
RMN 1H/CDCl3 : isomère E : 1.27(s,6H), 1.26à1.28(t,3H), 1.37(s,6H)_{,} 1.60à1.65(m,4H), 2.37(d,3H), 3.39(s,3H), 3.58(t,2H), 3.82(t,2H), 4.13à4.21(q,2H), 5.29(s,2H), 6.02(d,1H), 7.06(d,1H), 7.10(d,1H).
isomère Z : 1.25(s,6H), 1.20à1.25(t,3H), 1.35(s,6H), 1.63à1.65(m,4H), 2.40(d,3H), 3.38(s,3H), 3.58(t,2H), 3.82(t,2H), 4.09à4.12(q,2H), 5.28(s,2H), 5.44(d,1H), 7.08(d,1H), 7.11(d,1H).

### EXEMPLE 33

### Acide (Z)-5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoïque.

De manière analogue à l'exemple 2, à partir de 56 mg de l'isomère Z de l'exemple 32, on obtient 25 mg (48%) du composé attendu.
RMN 1H/CDCl3 : 1.25(s,6H), 1.39(s,6H), 1.60à1.68(m,4H), 2.41 (s,3H), 3.37(s,3H), 3.57(t,2H), 3.81(t,2H), 5.28(s,2H), 5.35(m,1H), 7.1(d, 2H)

### EXEMPLE 34

### Acide (E)-5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoïque.

De manière analogue à l'exemple 2, à partir de 155 mg dl'isomère E de l'exemple 32, on obtient 45 mg (32%) du composé attendu.
**RMN** 1H/CDCl3 : 1.26(s,6H), 1.37(s,6H), 1.60à1.67(m,4H), 2.38(d,3H), 3.39(s,3H), 3.61(t,2H), 3.84(t,2H), 5.30(s,2H), 6.10(d,1H), 7..3(d,1H), 7.15(d,1H)

### EXEMPLE 35

### 5-(3,5,5,8,8-Pentamethyl-5,6,7,8-tetrahydro-naphthalen-2-ylsulfanyl)-penta-2,4-diynoate de méthyle.

On ajoute goutte à goutte une solution à 70% d'éthylamine dans l'eau (563 ul) à un mélange de 3-bromopropynoate de methyle (632 mg, 2,88 mmol), de chlorhydrate d'hydroxylamine (162 mg, 2 ,33 mmol) et de chlorure de cuivre (I) (10 mg) dans le méthanol. Une solution de 6-éthynylsulfanyl-1,1,4,4,7-pentaméthyl-1,2,3,4-tetrahydro-naphtalène (exemple 7 (b))(500 mg, 1,94 mmol) dans le méthanol (5ml) est ensuite additionnée goutte à goutte. Le milieu réactionnel est agité 15 h. à température ambiante, traité par une solution saturée de chlorure d'ammonium dans l'eau et de l'acétate d'éthyle. La phase organique est lavée à l'eau, séchée sur sulfate de magnésium et concentré. Le produit est obtenu sous forme d'une huile après purification par chromatographie flash sur silice (heptane-CH₂Cl₂ 80/20).
RMN 1H/CDCl3: 1,26à1.28(s,12H);1.67(s,4H); 2.35(s,3H); 3.78(s,3H); 7.14(s,1H); 7.48(s,1H)
RMN 13C/CDCl3: 19.7;CH3/ 31.8;4*CH3/ 34.1;Cq/ 34.3;Cq/ 34.9;2*CH2/52.9;CH3/ 71.7;CH/ 72.1;CH/ 80.2;Cq/ 124.5;Cq/ 127.6;Cq/ 128.3;CH/129.3;CH/ 134.8;Cq/ 144.6;Cq/ 146.2;Cq/ 153.4;Cq

### EXEMPLE 36

### 5-(3-Benzyloxy-5,5,8,8-tetramethyl-56,7,8-tetrahydro-naphthalen-2-ylselanyl)-3-methyl-pent-2-en-4-ynoate d'ethyle.

### a) 3-benzyloxy-5,6,7,8-tetrahydro-5,5,8,8-tetramethylnaphtalene-2-disélénide

De manière analogue à l'exemple 20 b, le produit attendu est obtenu par action de césium carbonate sur le 3-hydroxy-5,6,7,8-tetrahydro-5,5,8,8-tetramethylnaphtalene-2-disélénide (exemple 15 b).

### c) 5-(3-Benzyloxy-5,5,8,8-tetramethyl-5,6,7,8-tetrahydro-naphthalen-2-ylselanyl)-3-methyl-pent-2-en-4-ynoate d'ethyle.

Le produit attendu est obtenu de manière analogue à l'exemple 22b, avec introduction du brome réalisée à -70°C. On obtient 650 mg (71%) d'une poudre jaunâtre
RMN 1H/CDCl3 : 1.23(s,6H), 1.26à1.32(s,t,9H), 1.67(s,4H), 2.40(d,3H), 4.14à4.22q,2H), 5.10(s,2H), 6.06(d,1H), 6.78(s,1H), 7.32à7.44(m,5H), 7.57(s,1H),

### EXEMPLE 37

### Acide 5-(3-Benzyloxy-5,5,8,8-tetramethyl-5,6,7,8-tetrahydro-naphthalen-2-ylselanyl)-3-methyl-pent-2-en-4-ynoïque.

De manière analogue à l'exemple 2, à partir de 630 mg du produit de l'exemple (36), on obtient le composé attendu sous forme d'une poudre jaunâtre.
RMN 1H/CDCl3 : 1.23(s,6H), 1.29(s,6H), 1.67(s,4H), 2.42(d,3H), 5.11(s,2H), 6.08(d,1H), 6.79(s,1H), 7.32à7.44(m,5H), 7.56(s,1H).

### EXEMPLE 38

### 5-[3-(2-methoxyethoxymethoxy)-5,5,8,8-tetramethyl-5,6,7,8-tetrahydro-naphthalen-2-ylselanyl]-3-methyl-pent-2-en-4-ynoate d'ethyle.

### a) 3-(2-methoxyethoxymethoxy)-5,6,7,8-tetrahydro-5,5,8,8-tetramethylnaphtalene-2-disélénide

De manière analogue à l'exemple 28 a, le produit attendu est obtenu par action dhydrure de sodium sur le 3-hydroxy-5,6,7,8-tetrahydro-5,5,8,8-tetramethylnaphtalene-2-disélénide (exemple 15 b).

### b) 5-[3-(2-methoxyethoxymethoxy)-5,5,8,8-tetramethyl-5,6,7,8-tetrahydro-naphthalen-2-ylselanyl]-3-methyl-pent-2-en-4-ynoate d'ethyle.

Le produit attendu est obtenu de manière analogue à l'exemple 22b, avec introduction du brome réalisée à -70°C. On obtient 705 mg (77%) d'une huile jaune
RMN 1H/CDCl3 : 1.27(s,6H), 1.25à1.28(t,3H), 1.37(s,6H), 1.62à1.63(m,4H), 2.35(d,3H), 3.39(s,3H), 3.58(t,2H), 3.83(t,2H), 4.13à4.21(q,2H), 5.29(s,2H), 6.03(d,1H), 7.16(s,2H).

### EXEMPLE 39

### Acide 5-[3-(2-methoxyethoxymethoxy)-5,5,8,8-tetramethyl-5,6,7,8-tetrahydro-naphthalen-2-ylselanyl]-3-methyl-pent-2-en-4-ynoïque.

De manière analogue à l'exemple 2, à partir de 693 mg du produit de l'exemple 38, on obtient le composé attendu sous forme d'une huile jaunâtre.
RMN 1H/CDCl3 : 1.27(s,6H), 1.37(s,6H), 1.62à1.63(m,4H), 2.36(d,3H), 3.39(s,3H), 3.60(t,2H), 3.84(t,2H), 5.30(s,2H), 6.09(d,1H), 7.14(d,1H), 7.19(d,1H).

### EXEMPLE 40

### 5-(3,5,5,8,8-Pentamethyl-5,6,7,8-tetrahydro-naphthalen-2-ylselanyl)-penta-2,4-diynoate de méthyle.

### a) 6-trimethylsilyléthynylselanyl-1,1,4,4-tetraméthyl-1,2,3,4-tetrahydro-naphtalène

De manière analogue à l'exemple 1c, le produit est obtenu par couplage de trimethylsilylacétylène (2,4 ml, 17,2 mmol) et de 1g (1,79 mmol) de 5,6,7,8-tetrahydro-3,5,5,8,8-pentaméthylnaphtalene-2-disélénide (exemple 3a) à température ambiante dans le DMF catalysé par Cul (1g).
RMN 1H/CDCl3: 0.10(s,9H); 1.10(d,12H); 1.48(s,4H); 2.09(s,3H); 6.90(s,1H); 7.54(s,1H)

### b) 6-éthynylselanyl-1,1,4,4-tetraméthyl-1,2,3,4-tetrahydro-naphtalène

De manière analogue à l'exemple 1b, le produit est obtenu par action de 1g de fluorure de césium sur le 6-trimethylsilyléthynylselanyl-1,1,4,4-tetraméthyl-1,2,3,4-tetrahydro-naphtalène (500 mg, 3,6 mmol) à température ambinate dans un mélange de méthanol et THF pendant 24h.
RMN 1H/CDCl3: 1.25(d,12H); 1.65(s,4H); 2.33(s,3H); 3.11(s,1H); 7.10(s,1H); 7.68(s,1H)
13C/CDCl3: 31.8;4*CH3/ 34.0;Cq/ 34.3;Cq/ 35.0;2*CH2/ 73.1;Cq/ 90.7;CH/124.5;Cq/128.5;CH/ 128.9;CH/ 134.5;Cq/ 144.6;Cq/ 144.9;Cq.

### d) 5-(3,5,5,8,8-Pentamethyl-5,6,7,8-tetrahydro-naphthalen-2-ylselanyl)-penta-2,4-diynoate de méthyle.

De manière analogue à l'exemple 35, le produit attendu est obtenu par couplage de 321 mg de bromopropynoate de methyle et 200 mg de 6-éthynylselanyl-1,1,4,4-tetraméthyl-1,2,3,4-tetrahydro-naphtalène avec un rendement de 28% (70 mg).
RMN 1H/CDCl3: 1.23(s,6H); 1.33(s,6H); 1.67(s,4H); 2.35(s,3H); 3.79(s,3H); 7.13(s,1H); 7.65(s,1H)

### EXEMPLE 41

### 5-(4-Benzyloxy-5,5,8,8-tetramethyl-5,6,7,8-tetrahydro-naphthalen-2-ylselanyl)-3-methyl-pent-2-en-4-ynoate d'ethyle

De manière analogue à l'exemple 22b, avec introduction de brome réalisée à -70°C, le produit attendu est obtenu par couplage de 464 mg de 4-benzyloxy-5,6,7,8-tetrahydro-5,5,8,8-tetramethylnaphtalene-2-disélénide (exemple 21b) et 300 mg de 3-méthyl-pent-2-en-4-ynoate d'éthyle avec un rendement de 65% (300 mg).
RMN 1H/CDCl3: 1.28(m,9H); 1.38(s,6H); 1.63(m,4H); 2.33(d,3H); 4.17(q,2H); 5.08(s,2H); 6.00(t,1H); 6.93(d,1H); 7.11(d,1H); 7.31à7.47(m,5H)
13C/CDCl3: 14.6;CH3/ 28.8;2*CH3/ 32.3;2*CH3/ 34.7;CH2/ 35.2;Cq/ 35.5;CH2/38.3;Cq60.4;CH2/ 70.9;CH2/ 78.0;Cq/ 105.9;Cq/ 110.9;CH/ 121.1;CH/123.0;Cq/ 125.5;Cq/ 128.0;5*CH/ 128.3;CH/ 129.0;4*CH/ 133.5;Cq/ 137.2;Cq/137.7;Cq/ 149.4;Cq/ 159.0;Cq/ 166.5;Cq

### EXEMPLE 42

### 5-[3-(5-Hydroxy-pentyloxy)-5,5,8,8-tetramethyl-5,6,7,8-tetrahydro-naphthalen-2-ylselanyl]-3-methyl-pent-2-en-4-ynoate d'éthyle.

Un mélange du produit de l'exemple 43 (105 mg, 177 umol), d'une solution 1M dans le THF de fluorure de tétra-n-butylammonium (770 ul) dans le THF est agité à température ambiante 6h. Le milieu réactionnel est traité par une solution d'HCl 1N et de l'éther éthylique. Après décantation la phase organique est lavée à l'eau, séchée sur sulfate de magnésium anhydre et concentrée. Le produit est purifié par cristallisation dans un mélange heptane, éther éthylique. Masse : 48 mg, poudre jaunâtre. Tf=134°C.
RMN 1H/CDCl3: 1.18 à 1.29 (m, 14H) ; 1.56 à 1.68 (m, 8H); 1.83 (m,2H); 2.42 (d,3H); 3.69 (t,2H); 4.02 (t,2H); 6.08 (d, 1H) ; 6.72 (s, 1H) ; 7.54 (s, 1H).

### EXEMPLE 43

### Acide 5-(4-Fluoro-3-methyl-phenylselanyl)-3-methyl-pent-2-en-4-ynoïque.

### a) Préparation de la résine.

Un mélange de 3-methyl-pent-2-en-4-ynoic acid (exemple 20 c) (12,6 mmol, 1,51 g), de carbonate de césium (16,8 mmol, 3,24 g) et 4g de résine Wang-bromo polystyrene (novabiochem ref 01-64-0186) dans 30 ml de DMF est chauffée à 60°C pendant 24h. Le mélange est filtré puis lavé trois fois par du DMF, de l'eau, du THF du méthanol et du dichlorométhane.

### b) 4-fluoro-3-methyl-phenyl disélénide.

Le produit attendu est synthétisé selon le protocole opératoire de l'exemple 22 a.

### c) bromure de 4-fluoro-3-methyl-phenylselanyl.

Du brome (0,048 ml, 0,93 mmol), est additionné à une solution de 4-Fluoro-3-methyl-phenyl disélénide (0,94 mmol) dans le THF (1 ml). Le mélange est agité à température ambiante 2 h, puis le solvant est éliminé par un fort courant d'azote.

### d) Acide 5-(4-fluoro-3-methyl-phenylselanyl)-3-methyl-pent-2-en-4-ynoïque.

Un mélange de la résine précédemment obtenue (1g, 1,04 mmol) de bromure de 4-fluoro-3-methyl-phenylselanyl (2,5 mmol) de Cul (I) (5mmol) de tributylamine (4 mmol), est agité à 60°C pendant 24h. La résine est filtrée puis lavée trois fois par du DMF, de l'eau, du THF et du dichlorométhane. Le produit est obtenu après clivage par une solution d'acide trifluoroacétique à 10% pendant 7mn.
RMN 1H/CDCl3: 2.29 (d,3H); 2,31 (d, 3H) ; 6.05 (d, 1H) ; 7.13(d, 1H) ; 7.47 à 7.54 (m, 2H).

### EXEMPLE 44

### Acide 3-Methyl-5-p-tolylselanyl-pent-2-en-4-ynoïque.

Le produit attendu est obtenu selon le même protocole opératoire que l'exemple 45.
RMN 1H/CDCl3: 2.34 (s,3H); 6.04 (s, 1H) ; 7.16 (d, 2H) ; 7.42 (d, 2H).

### EXEMPLE 45

### Acide 5-(6-bromo-4,4-Dimethyl-thiochroman-8-ylselanyl)-3-methyl-pent-2-en-4-ynoïque.

### (a) 2-bromo-1-(3-méthylbut-2-ènylthio)benzène.

Dans un tricol, on introduit 19,30 g (102,0 mmoles) de 2-bromothiophénol, 160 ml de DMF et 15,50 g (112,0 mmoles) de carbonate de potassium. On ajoute goutte à goutte 13 ml (112,0 mmoles) de 1-bromo-3-méthyl-2-butène et agite à la température ambiante pendant deux heures. On verse le milieu réactionnel dans l'eau, extrait avec de l'acétate d'éthyle, décante la phase organique, lave à l'eau, sèche sur sulfate de magnésium, évapore. On recueille 26,00 g (99%) du composé attendu, sous la forme d'une huile orangée.
¹H NMR (CDCl₃) d 1,65 (s, 3H), 1,73 (s, 3H), 3,56 (d, 2H, J = 7,7 Hz), 5,32 (td, 1H, J = 7,7 / 1,4 Hz), 6,96 à 7,06 (m, 1H), 7,22 à 7,26 (m, 2H), 7,52 (d, 1H, J = 7,7 Hz).

### (b) 4,4-diméthyl-8-bromothiochromane.

Dans un tricol, on introduit 26,00 g (102,0 mmoles) de 2-bromo-1-(3-méthylbut-2-ènyithio)benzène, 180 ml de toluène et 23,20 g (122,0 mmoles) d'acide para-toluène sulfonique. On chauffe à reflux pendant quatre heures et évapore le milieu réactionnel à sec. On reprend par une solution aqueuse d'hydrogénocarbonate de sodium, extrait avec de l'acétate d'éthyle, décante la phase organique, sèche sur sulfate de magnésium, évapore. Le résidu obtenu est purifié par chromatographie sur colonne de silice, élué avec de l'heptane. On recueille 20 g (76%) du composé attendu, sous la forme d'une huile orangée.
¹H NMR (CDCl₃) d 1,33 (s, 6H), 1,94 (t, 2H, J = 6,0 Hz), 3,04 (t, 2H, J = 6,1 Hz), 6,89 (t, 1H, J = 7,9 Hz), 7,34 (d, 2H, J = 7,9 Hz).

### (c) 4,4-Dimethyl-thiochroman-8-disélénide.

Un cristal d'iode, du magnésium (208 mg, 8,56 mmol) et quelques gouttes d'une solution de 4,4-diméthyl-8-bromothiochromane (2g, 7,78 mmol) dans l'éther éthylique (15ml) sont chauffés jusqu'à l'amorçage de l'organomagnésien. Le reste de la solution est alors additionnée goutte à goutte. Le milieu réactionnel est chauffé 2h, puis le sélénium (615 mg, 7,78 mmol) est additionné à température ambiante. L'agitation est poursuivie 30 mn puis, une solution d'HCI 1N est additionnée. Le mélange réactionnel est traité par de l'éther éthylique. La phase organique est lavée 2 fois à l'eau, séchée sur sulfate de magnésium anhydre et concentrée à l'évaporateur rotatif sous vide à 40°C. De l'éthanol et de l'hydroxyde de sodium sont additionnés à l'huile obtenue. Le mélange est agité vigoureusement quelques minutes, puis est concentré à l'évaporateur rotatif sous vide à 40°C.
Le produit est purifié sur colonne de silice (dichlorométhane 20 - heptane 80).
Solide blanc. Masse 300 mg. Rendement : 15%.
RMN 1H (CDCl₃) : 1,33 (6H, s), 1,96 (2H, m), 3,09 (2H, m), 6,93 (1H Ar, t, J=7,8 Hz), 7,26 (1H Ar, dd, J=7,8 Hz, J=1,3 Hz), 7,47 (1H Ar, dd, J=7,8 Hz, J=1,3 Hz).

### d) Acide 5-(6-bromo-4,4-Dimethyl-thiochroman-8-ylselanyl)-3-methyl-pent-2-en-4-ynoïque.

Le protocole opératoire est identique à celui suivi dans l'exemple 45 en utilisant le sélénide précédemment obtenu.
RMN 1H (CDCl₃) : 1,34 (6H, s), 1,96 (2H, m), 2,40 (3H, s), 3,13 (2H, m), 6,13 (1H, s), 7,43 (1H Ar, d), 7,64 (1H Ar, d).

### EXEMPLE 46

Dans cet exemple on a illustré plusieurs résultats de tests biologiques d'exmples de composé de l'invention.

### ACTIVITE BIOLOGIQUE

| **Produit** | **IC 50 (Nm)**^{**a**} | **% d'inhibition**^{**b**} |
|---|---|---|
| EXEMPLE 10 | 671 | 57% |
| EXEMPLE 12 | 71 | 92% |
| EXEMPLE 14 | 471 | 61% |

### a) Détermination de l'IC50 des composés antagonistes

L'activité antagoniste peut aussi être caractérisée dans ce système de cotransfection par la détermination de la dose nécessaire pour atteindre 50% de l'inhibition de l'activité agoniste obtenue avec 3 nM de 6-[1-(3,5,5,8,8-Pentamethyl-5,6,7,8-tetrahydro-naphthalen-2-yl)-cyclopropyl]-nicotinic acid (IC50). Le produit est alors testé à 5 concentrations différentes variant de 10-5 à 10-9 M final en présence de l'agoniste 6-[1-(3,5,5,8,8-Pentamethyl-5,6,7,8-tetrahydro-naphthalen-2-yl)-cyclopropyl]-nicotinic acid 3nM La courbe dose réponse obtenue permet de déterminer l'IC50 propre au composé.

### b) Test de transactivation antagoniste RXR

Les propriétés d'inhibition de l'activation des éléments de réponse RXR (RXRE) peuvent être déterminées par des méthodes reconnues dans l'art.
Le plasmide d'expression du récepteur pSG5-RXRalpha, et le plasmide reporter CRBPII-tk-Luc sont introduits dans la lignée de cellules de reins de singe Cos7 par la méthode de coprécipitation au phosphate de calcium décrite dans Molecular Cloning: A laboratory manual (Sambrook et al. eds. Cold Spring harbor Lab. Publ. 1989). 18 heures plus tard, les cellules sont rincées au PBS et sont placées en milieu DMEM sans rouge de phénol (Gibco-BRL) contenant 10% de sérum délipidé (Jacques Boy) et 3 nM de l'agoniste de référence 6-[1-(3,5,5,8,8-Pentamethyl-5,6,7,8-tetrahydro-naphthalen-2-yl)-cyclopropyl]-nicotinic acid. Le composé test est ajouté à 10-6M final. Après 18h de traitement, les cellules sont rincées avec du PBS et lysées dans 0.1M K₃PO₄ (pH 7.8), 1.0% TRITON X100, 1.0 mM DTT, 1mM EDTA. L'activité luciférase des lysats cellulaires est mesurée comme décrit par deWet et al. dans Mol. Cell. Biol. 7:725 (1987). Les résultats sont exprimés en pourcentage d'inhibition de l'induction obtenue avec l'agoniste 6-[1-(3,5,5,8,8-Pentamethyl-5,6,7,8-tetrahydro-naphthalen-2-yl)-cyclopropyl]-nicotinic acid seul.

Les résultats présentés ci-dessus montrent l'activité antagoniste RXR de plusieurs exemples de composés de l'invention.

### EXEMPLE 47

Dans cet exemple, on a illustré diverses formulations concrètes à base des composés selon l'invention.

### A- VOIE ORALE

(a) Comprimé de 0,2 g
   - Composé de l'exemple 1 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé de l'exemple 3 0,001 g
   - Glycérine 0,500 g
   - Sorbitol à 70% 0,500 g
   - Saccharinate de sodium 0,010 g
   - Parahydroxybenzoate de méthyle 0,040 g
   - Arôme qs
   - Eau purifiée qsp 5 ml
(c) Comprimé de 0,8 g
   - Composé de l'exemple 15 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé de l'exemple 36 0,05 g
   - Glycérine 1,000g
   - Sorbitol à 70% 1,000g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé de l'exemple 27 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 8 0,300 g
   - Vaseline blanche codex 100 g
(c) Crème Eau-dans-Huile non ionique
   - Composé de l'exemple 41 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé de l'exemple 6 0,100 g
   - Polyéthylène glycol (PEG 400) 69,900 g
   - Ethanol à 95% 30,000 g
(e) Onguent hydrophobe
   - Composé de l'exemple 19 0,300 g
   - Mirystate d'isopropyle 36,400 g
   - Huile de silicone ("Rhodorsil 47 V 300" vendu par RHONE-POULENC) 36,400 g
   - Cire d'abeille 13,600 g
   - Huile de silicone ("Abil 300.000 cst" vendu par GOLDSCHMIDT) 100g
(f) Crème Huile-dans-Eau non ionique
   - Composé de l'exemple 4 0,500 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile 100 g

## Revendications

1. Composés hétéroéthynylénés, **caractérisés par le fait qu'**ils répondent à la formule générale (I) suivante : 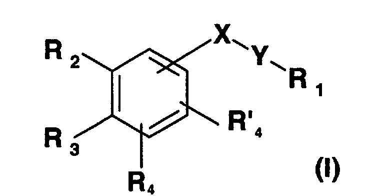 dans laquelle:
- R₁ représente :
(i) le radical -CH3
(ii) le radical -CH2-O-R₅
(iii) le radical -COR₆
R₅ et R₆ ayant les significations données ci-après,
- X représente : O, Se , S(O)n, n étant 0, 1 ou 2,
- Y représente un radical divalent qui a pour formule :
a) 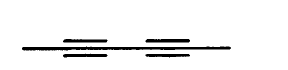
b) 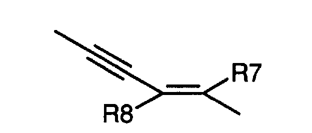
R₇, et R₈ ayant les significations données ci-après,
- R₂ et R₃ pris ensemble forment avec le cycle aromatique adjacent un cycle à 5 ou 6 chaînons éventuellement substitué par des groupes méthyle et / ou éventuellement interrompu par un atome d'oxygène ou de soufre,
sachant que R₂ et R₃ indépendamment identiques ou différents peuvent représenter :
a) un atome d'hydrogène,
b) un radical choisi parmi les radicaux
- méthyl
- tertiobutyl,
- 1-méthylcyclohexyl,
- 1-Adamantyl,
c) un radical -OR₉
d) un radical polyéther
e) un atome d'halogène
R₉ ayant la signification donnée ci-après,
étant entendu que au moins un des substituants R₂ et R₃ représente (b),
- R₄ représente un atome d'hydrogène, un radical alkyle de 1 à 6 atomes de carbone, un radical OR₁₀, un radical polyéther un radical COR₁₁, ou un atome d'halogène,
R₁₀ et R₁₁ ayant les significations données ci-après,
- R'₄ représente un atome d'hydrogène ou un atome d'halogène,
- R₅ représente un atome d'hydrogène ou un radical alkyle de 1 à 6 atomes de carbone,
- R₆ représente :
(i) un atome d'hydrogène
(ii) un radical alkyle de 1 à 6 atomes de carbone
(iii) le radical OR₁₂
R₁₂ ayant la signification donnée ci-après,
(iv) le radical de formule 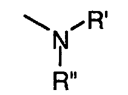 R' et R" ayant les significations données ci-après,
- R₇ et R₈ identiques ou différents, représentent un atome d'hydrogène un radical alkyle de 1 à 6 atomes de carbone, ou un radical aryle,
- -R₉ et R₁₀, identiques ou différents, représentent un atome d'hydrogène, un radical alkyle de 1 à 6 atomes de carbone, un radical aryle éventuellement substitué, un radical aralkyle éventuellement substitué, un radical monohydroxyalkyle ou polyhydroxyalkyle ou un radical acyle de 1 à 4 atomes de carbone,
- R₁₁ représente un radical alkyle de 1 à 6 atomes de carbone, un radical OR₁₃ ou un radical 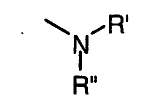 R₁₃, R' et R" ayant les significations données ci-après,
- R₁₂ et R₁₃ identiques ou différents représentent un atome d'hydrogène, un radical alkyle de 1 à 6 atomes de carbone, un radical aryle, un radical aralkyle éventuellement substitué, un radical monohydroxyalkyle ou un radical polyhydroxyalkyle,
- R' et R", identiques ou différents, représentent un atome d'hydrogène, un radical alkyle de 1 à 6 atomes de carbone, un radical mono ou polyhydroxyalkyle, un radical aryle eventuellement substitué ou un reste d'amino acide, ou encore pris ensemble avec l'atome d'azote forment un hétérocycle,
ainsi que leurs sels et leurs isomères optiques et géométriques.

2. Composés selon la revendication 1, **caractérisés par le fait qu'**ils se présentent sous forme de sels d'un métal alcalin ou alcalino-terreux, ou encore de zinc ou d'une amine organique.

3. Composés selon l'une des revendications 1 ou 2, **caractérisés par le fait que** les radicaux alkyles de 1 à 6 atomes de carbone sont choisis parmi le groupe constitué par les radicaux méthyle, éthyle, isopropyle, butyle et tertiobutyle.

4. Composés selon l'une des revendications précédentes, **caractérisés en ce que** les radicaux monohydroxyalkyles sont choisis parmi le groupe constitué par les radicaux 2-hydroxyéthyle, 2-hydroxypropyle et 3-hydroxypropyle.

5. Composés selon l'une des revendications précédentes, **caractérisés en ce que** les radicaux polyhydroxyalkyles sont choisis parmi le groupe constitué par les radicaux 2,3-dihydroxypropyle, 2,3,4-trihydroxybutyle, 2,3,4,5-tétrahydroxypentyle ou le reste du pentaérythritol.

6. Composés selon l'une des revendications précédentes, **caractérisés en ce que** le radical aryle est un radical phényle éventuellement substitué par au moins un atome d'halogène, un radical hydroxyle ou une fonction nitro.

7. Composés selon l'une des revendications précédentes, **caractérisés en ce que** les radicaux aralkyles sont choisis parmi le groupe constitué par les radicaux benzyle ou phénéthyle, éventuellement substitués par au moins un atome d'halogène, un hydroxyle ou une fonction nitro.

8. Composés selon l'une quelconque des revendications précédentes, **caractérisés en ce que** les restes d'aminoacide sont choisis dans le groupe constitué par les restes dérivant de l'un des 20 aminoacides de configuration L ou D constitutifs de protéines de mammifères.

9. Composés selon l'une quelconque des revendications précédentes, **caractérisés en ce que** les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

10. Composés selon l'une quelconque des revendications précédentes, **caractérisés en ce que** les radicaux polyéther sont choisis parmi les radicaux méthoxyméthyl éther, méthoxyéthoxyméthyl éther ou méthylthiométhyl éther.

11. Composés selon la revendication 1, **caractérisés par le fait qu'**ils sont pris, seuls ou en mélanges, dans le groupe constitué par :
3-Methyl-5-(5,5,8,8-tetramethyl-5,6,7,8-tetrahydro-naphtalen-2-ylselanyl)-pent-2-en-4-ynoate d'éthyle
Acide 3-méthyl-5-(5,5,8,8-tetraméthyl-5,6,7,8,-tetrahydro-naphtalen-2-ylselanyl)-pent-2-en-4-ynoique
3-méthyl-5-(3,5,5,8,8-pentaméthyl-5,6,7,8-tetrahydro-naphtalen-2-ylselanyl)-pent-2-en-4-ynoate d'éthyle
Acide 3-méthyl-5-(3,5,5,8,8-pentaméthyl-5,6,7,8-tetrahydro-naphtalen-2-ylselanyl)-pent-2-en-4-ynoique
3-méthyl-5-(5,5,8,8-tetraméthyl-5,6,7,8-tetrahydro-naphtalen-2-ylsulfanyl)-pent-2-en-4-ynoate d'éthyle
Acide 3-méthyl-5-(5,5,8,8-tetraméthyl-5,6,7,8-tetrahydro-naphtalen-2-ylsulfanyl)-pent-2-en-4-ynoique
3-méthyl-5-(3,5,5,8,8-pentaméthyl-5,6,7,8-tetrahydro-naphtalen-2-ylsulfanyl)-pent-2-en-4-ynoate d'éthyle
Acide 3-méthyl-5-(3,5,5,8,8-pentaméthyl-5,6,7,8-tetrahydro-naphtalen-2-ylsulfanyl)-pent-2-en-4-ynoique
3-Methyl-5-(5,5,8,8-tetramethyl-3-propoxy-5,6,7,8-tetrahydro-naphthalen-2-ylselanyl)-pent-2-en-4-ynoate d'éthyle
Acide 3-Methyl-5-(5,5,8,8-tetramethyl-3-propoxy-5,6,7,8-tetrahydro-naphthalen-2-ylselanyl)-pent-2-en-4-ynoïque
3-Methyl-5-(5,5,8,8-tetramethyl-4-propoxy-5,6,7,8-tetrahydro-naphthalen-2-ylselanyl)-peut-2-en-4-ynoate d'éthyle
Acide 3-Methyl-5-(5,5,8,8-tetramethyl-4-propoxy-5,6,7,8-tetrahydro-naphthalen-2-ylselanyl)-pent-2-en-4-ynoïque
5-(4-Methoxymethoxy-5,5,8,8-tetramethyl-5,6,7,8-tetrahydro-naphthalen-2-ylselanyl)-3-methyl-pent-2-en-4-ynoate d'éthyle
Acide 5-(4-Methoxymethoxy-5,5,8,8-tetramethyl-5,6,7,8-tetrahydro-naphthalen-2-ylselanyl)-3-methyl-pent-2-en-4-ynoïque
5-(3-Hydroxy-5,5,8,8-tetramethyl-5,6,7,8-tetrahydro-naphthalen-2-ylselanyl)-3-methyl-pent-2-en-4-ynoate d'éthyle
Acide 5-(3,5,5,8,8-Pentamethyl-5,6,7,8-tetrahydro-naphthalen-2-ylselanyl)-3-phenyl-pent-2-en-4-ynoïque
Acide 5-(4-Methoxymethoxy-5,5,8,8-tetramethyl-5,6,7,8-tetrahydro-naphthalen-2-ylselanyl)-3-phenyl-pent-2-en-4-ynoïque
5-(4-Hydroxy-5,5,8,8-tetramethyl-5,6,7,8-tetrahydro-naphthalen-2-ylselanyl)-3-phenyl-pent-2-en-4-ynoate de méthyle
Acide 3-propyl-5-(5,5,8,8-tetraméthyl-5,6,7,8,-tetrahydro-naphtalen-2-ylselanyl)-pent-2-en-4-ynoique
Acide 5-(4-Benzyloxy-3-bromo-5,5,8,8-tetramethyl-5,6,7,8-tetrahydro-naphthalen-2-ylselanyl)-3-methyl-pent-2-en-4-ynoïque
Acide 5-(4-Benzyloxy-5,5,8,8-tetramethyl-5,6,7,8-tetrahydro-naphthalen-2-ylselanyl)-3-phenyl-pent-2-en-4-ynoïque
5-[5-Adamantan-1-yl-4-(2-methoxy-ethoxymethoxy)-2-methyl-phenylselanyl]-3-methyl-pent-2-en-4-ynoate d'éthyle
Acide 5-[5-Adamantan-1-yl-4-(2-methoxy-ethoxymethoxy)-2-methyl-phenylselanyl]-3-methyl-pent-2-en-4-ynoïque
Acide 5-[5-Adamantan-1-yl-4-(2-methoxy-ethoxymethoxy)-2-methyl-phenylselanyl]-3-propyl-pent-2-en-4-ynoïque
Acide 5-[5-Adamantan-1-yl-4-(2-methoxy-ethoxymethoxy)-2-methyl-phenylselanyl]-3-phenyl-pent-2-en-4-ynoïque
5-(3,5-Di-tert-butyl-2-methoxymethoxy-phenylselanyl)-3-methyl-pent-2-en-4-ynoate d'éthyle
Acide 5-(3,5-Di-*tert*-butyl-2-methoxymethoxy-phenylselanyl)-3-methyl-pent-2-en-4-ynoïque
3-Methyl-5-(5,5,8,8-tetramethyl-3-hexyloxy-5,6,7,8-tetrahydro-naphthalen-2-ylselanyl)-pent-2-en-4-ynoate d' éthyle
Acide 3-Methyl-5-(5,5,8,8-tetramethyl-3-hexyloxy-5,6,7,8-tetrahydro-naphthalen-2-ylselanyl)-pent-2-en-4-ynoïque
Acide 5-[4-Adamantan-1-yl-3-(2-methoxy-ethoxymethoxy)-phenylselanyl]-3-propyl-pent-2-en-4-ynoïque
5-[2-(adamantan-1-yl)-1-méthoxyéthoxyméthoxyphen-5-ylselanyl)-3-methyl-pent-2-en-4-ynoate d'éthyle
5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoate d'éthyle
Acide (Z)-5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoïque
Acide (E)-5-[4-(2-Methoxy-ethoxymethoxy)-5,5,8,8-tetramethyl-5,6,7,8-tetrahydro-naphthalen-2-ylsulfanyl]-3-methyl-pent-2-en-4-ynoïque
5-(3,5,5,8,8-Pentamethyl-5,6,7,8-tetrahydro-naphthalen-2-ylsulfanyl)-penta-2,4-diynoate de méthyle
5-(3-Benzyloxy-5,5,8,8-tetramethyl-5,6,7,8-tetrahydro-naphthalen-2-ylselanyl)-3-methyl-pent-2-en-4-ynoate d'ethyle
Acide 5-(3-Benzyloxy-5,5,8,8-tetramethyl-5,6,7,8-tetrahydro-naphthalen-2-ylselanyl)-3-methyl-pent-2-en-4-ynoïque
5-[3-(2-methoxyethoxymethoxy)-5,5,8,8-tetramethyl-5,6,7,8-tetrahydro-naphthalen-2-ylselanyl]-3-methyl-pont-2-en-4-ynoate d'ethyle
Acide 5-[3-(2-methoxyethoxymethoxy)-5,5,8,8-tetramethyl-5,6,7,8-tetrahydro-naphthalen-2-ylselanyl]-3-methyl-pent-2-en-4-ynoïque
5-(3,5,5,8,8-Pentamethyl-5,6,7,8-tetrahydro-naphthalen-2-ylselanyl)-penta-2,4-diynoate de méthyle
5-(4-Benzyloxy-5,5,8,8-tetramethyl-5,6,7,8-tetrahydro-naphthalen-2-ylselanyl)-3-methyl-pent-2-en-4-ynoate d'ethyle
5-[3-(5-Hydroxy-pentyloxy)-5,5,8,8-tetramethyl-5,6,7,8-tetrahydro-naphthalen-2-ylselanyl]-3-methyl-pent-2-en-4-ynoate d'éthyle
Acide 5-(4-Fluoro-3-methyl-phenylselanyl)-3-methyl-pent-2-en-4-ynoïque
Acide 3-Methyl-5-p-tolylselanyl-pent-2-en-4-ynoïque
Acide 5-(6-bromo-4,4-Dimethyl-thiochroman-8-ylselanyl)-3-methyl-pent-2-en-4-ynoique

12. Composés selon la revendication 1, **caractérisés par le fait qu'**ils présentent au moins l'une des, et de préférence toutes les, caractéristiques suivantes :
- R₁ représente le radical -COR₆;
- X représente le radical Se ou S;
- le groupement -X-Y-R1 est en position para par rapport au substituant R3 sur le cycle aromatique;
- R₂ et R₃ pris ensemble forment avec le cycle aromatique adjacent un cycle à 5 ou 6 chaînons éventuellement substitué par des groupes méthyle et / ou éventuellement interrompu par un atome d'oxygène ou de soufre, ou R₂ ou R₃ est un radical 1-Adamantyl.

13. Composés selon l'une quelconque des revendications précédentes pour une utilisation comme médicament.

14. Composés selon la revendication 13 pour une utilisation comme médicament destiné au traitement des affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle; pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation; pour traiter toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; pour traiter d'autres désordres dermatologiques tels que les dermatoses bulleuses et les maladies du collagène; pour traiter certains troubles ophtalmologiques, notamment les cornéopathies; pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique; pour prévenir ou guérir les stigmates de l'atrophie épidermique et/ou dermique induite par les corticostéroïdes locaux ou systémiques, ou tout autre forme d'atrophie cutanée, pour prévenir ou traiter les troubles de la cicatrisation ou pour prévenir ou réparer les vergetures; pour favoriser la cicatrisation, pour lutter contre les troubles de la fonction sébacée tels que l'hyperséborrhée de l'acné ou la séborrhée simple; pour le traitement ou la prévention des états cancéreux ou précancéreux, plus particuliérement les leucémies promyélocytaires; pour le traitement d'affections inflammatoires telles que l'arthrite, pour le traitement de toute affection d'origine virale au niveau cutané ou général; pour la prévention ou le traitement de l'alopécie; pour le traitement d'affections dermatologiques à composante immunitaire; pour le traitement d'affections du système cardiovasculaire telles que l'artériosclérose, l'hypertension, le diabète non-insulino dépendant ainsi que l'obésité, pour le traitement de désordres cutanés dus à une exposition aux rayonnements U.V..

15. Composition pharmaceutique, **caractérisée par le fait qu'**elle comprend, dans un support pharmaceutiquement acceptable, au moins l'un des composés tels que définis à l'une quelconque des revendications 1 à 12.

16. Composition selon la revendication 15, **caractérisée en ce que** la concentration en composé(s) selon l'une des revendications 1 à 12 est comprise entre 0,001 % et 5 % en poids par rapport à l'ensemble de la composition.

17. Composition cosmétique, **caractérisée par le fait qu'**elle comprend, dans un support cosmétiquement acceptable, au moins l'un des composés tels que définis à l'une quelconque des revendications 1 à 12.

18. Composition selon la revendication 17, **caractérisée en ce que** la concentration en composé(s) selon l'une des revendications 1 à 12 est comprise entre 0,001 % et 3 % en poids par rapport à l'ensemble de la composition.

19. Utilisation d'une composition cosmétique telle que définie à l'une des revendications 17 ou 18 pour l'hygiène corporelle ou capillaire.

## Patentansprüche

1. Heteroethinylenverbindungen, **dadurch gekennzeichnet, daß** sie der folgenden allgemeinen Formel (I) entsprechen: 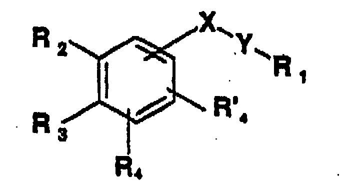 worin bedeuten:
- R₁
(i) die Gruppe -CH₃,
(ii) eine Gruppe -CH₂-O-R₅,
(iii) eine Gruppe -COR₆,
wobei R₅ und R₆ die nachfolgend angegebenen Bedeutungen aufweisen;
- X: O, Se, S(O)ₙ, wobei n 0, 1 oder 2 bedeutet;
- Y eine zweiwertige Gruppe der Formel:
a) 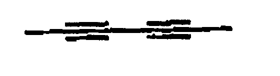
b) 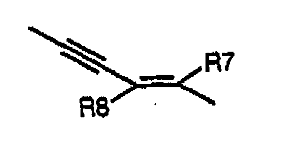
wobei R₇ und R₈ die nachfolgend angegebenen Bedeutungen aufweisen;
- R₂ und R₃ bilden mit dem angrenzenden aromatischen Ring einen 5- oder 6-gliedrigen Ring, der gegebenenfalls mit Methyl substituiert und/oder gegebenenfalls durch ein Sauerstoffoder Schwefelatom unterbrochen ist;
- wobei R₂ und R₃, die identisch oder voneinander verschieden sind, unabhängig bedeuten können:
a) Wasserstoff;
b) eine Gruppe, die ausgewählt ist unter:
- Methyl,
- *t*-Butyl,
- 1-Methylcyclohexyl,
- 1-Adamantyl;
c) -OR₉;
d) eine Polyethergruppe;
e) ein Halogenatom;
wobei R₉ die nachstehend angegebene Bedeutung aufweist; mit der Maßgabe, daß mindestens ein Substituent R₂ oder R₃ (b) bedeutet;
- R₄ ein Wasserstoffatom, eine Alkylgruppe mit 1 bis 6 Kohlenstoffatomen, eine Gruppe OR₁₀, eine Polyethergruppe, eine Gruppe COR₁₁ oder ein Halogenatom, wobei R₁₀ und R₁₁ die nachfolgend angegebenen Bedeutungen aufweisen;
- R₄' ein Wasserstoffatom oder ein Halogenatom;
- R₅ ein Wasserstoffatom oder eine Alkylgruppe mit 1 bis 6 Kohlenstoffatomen;
- R₆:
(i) ein Wasserstoffatom»
(ii) eine Alkylgruppe mit 1 bis 6 Kohlenstoffatomen,
(iii) eine Gruppe OR₁₂, wobei R₁₂ die nachfolgend angegebenen Bedeutungen aufweist;
(iv) eine Gruppe der Formel: 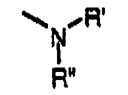 in der R' und R" die nachfolgend angegebenen Bedeutungen aufweisen;
- R₇ und R₈, die identisch oder voneinander verschieden sind, ein Wasserstoffatom, eine Alkylgruppe mit 1 bis 6 Kohlenstoffatomen oder eine Arylgruppe;
- R₉ und R₁₀, die identisch oder voneinander verschieden sind, ein Wasserstoffatom, eine Alkylgruppe mit 1 bis 6 Kohlenstoffatomen, eine gegebenenfalls substituierte Arylgruppe, eine gegebenenfalls substituierte Aralkylgruppe, eine Monooder Polyhydroxyalkylgruppe oder eine Acylgruppe mit 1 bis 4 Kohlenstoffatomen;
- R₁₁ eine niedere Alkylgruppe, eine Gruppe OR₁₃ oder eine Gruppe: 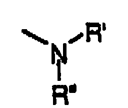 in der R' und R" die nachfolgend angegebenen Bedeutungen aufweisen;
- R₁₂ und R₁₃, die identisch oder voneinander verschieden sind, ein Wasserstoffatom, eine Alkylgruppe mit 1 bis 6 Kohlenstoffatomen, eine Arylgruppe, eine gegebenenfalls substituierte Aralkylgruppe, eine Monohydroxyalkylgruppe oder eine Polyhydroxyalkylgruppe;
- R' und R", die identisch oder voneinander verschieden sind, ein Wasserstoffatom, eine Alkylgruppe mit 1 bis 6 Kohlenstoffatomen, eine Mono- oder Polyhydroxyalkylgruppe, eine gegebenenfalls substituierte Arylgruppe oder einen Aminosäurerest oder die Gruppen R' und R" bilden gemeinsam einen Heterocyclus,
sowie ihre Salze und optischen und geometrischen Isomere.

2. Verbindungen nach Anspruch 1, **dadurch gekennzeichnet, daß** sie in Form von Alkalimetallsalzen, Erdalkalimetallsalzen, Zinksalzen oder Salzen von organischen Aminen vorliegen.

3. Verbindungen nach einem der Ansprüche 1 oder 2, **dadurch gekennzeichnet, daß** die Alkylgruppen mit 1 bis 6 Kohlenstoffatomen unter Methyl, Ethyl, Isopropyl, Butyl und *t*-Butyl ausgewählt sind.

4. Verbindungen nach einem der vorhergehenden Ansprüche, **dadurch gekennzeichnet, daß** die Monohydroxyalkylgruppen unter 2-Hydroxyethyl, 2-Hydroxypropyl und 3-Hydroxypropyl ausgewählt sind.

5. Verbindungen nach einem der vorhergehenden Ansprüche, **dadurch gekennzeichnet, daß** die Polyhydroxyalkylgruppen unter 2,3-Dihydroxypropyl, 2,3,4-Trihydroxybutyl, 2,3,4,5-Tetrahydroxypentyl oder Pentaerythrit ausgewählt sind.

6. Verbindungen nach einem der vorhergehenden Ansprüche, **dadurch gekennzeichnet, daß** die Arylgruppe Phenyl bedeutet, wobei die Phenylgruppe gegebenenfalls mit mindestens einem Halogenatom, einer Hydroxygruppe oder einer Nitrogruppe substituiert ist.

7. Verbindungen nach einem der vorhergehenden Ansprüche, **dadurch gekennzeichnet, daß** die Aralkylgruppen unter Benzyl oder Phenethyl ausgewählt sind, wobei diese Gruppen gegebenenfalls mit mindestens einem Halogenatom, einer Hydroxygruppe oder einer Nitrogruppe substituiert sind.

8. Verbindungen nach einem der vorhergehenden Ansprüche, **dadurch gekennzeichnet, daß** die Aminosäurereste unter den Resten ausgewählt sind, die von einer der 20 Aminosäuren mit L-oder D-Konfiguration abgeleitet sind, die die Proteine der Säuger aufbauen.

9. Verbindungen nach einem der vorhergehenden Ansprüche, **dadurch gekennzeichnet, daß** die heterocyclischen Gruppen unter den Gruppen Piperidino, Morpholino, Pyrrolidino oer Piperazino ausgewählt sind, die gegebenenfalls in 4-Stellung mit einer C₁₋₆-Alkylgruppe oder einer Mono- oder Polyhydroxyalkylgruppe substituiert sind.

10. Verbindungen nach einem der vorhergehenden Ansprüche, **dadurch gekennzeichnet, daß** die Polyethergruppen unter Methoxymethylether, Methoxyethoxymethylether oder Methylthiomethylether ausgewählt sind.

11. Verbindungen nach Anspruch 1, **dadurch gekennzeichnet, daß** sie unter den folgenden Verbindungen oder deren Gemischen ausgewählt sind:
Ethyl-3-methyl-5-(5,5,8,8-tetramethyl-5,6,7,8-tetrahydronaphthalin-2-ylselanyl)-pent-2-en-4-inoat,
3-Methyl-5-(5,5,8,8-tetramethyl-5,6,7,8-tetrahydro-naphthalin-2-ylselanyl)-pent-2-en-4-in-carbonsäure,
Ethyl-3-methyl-5-(3,5,5,8,8-pentamethyl-5,6,7,8-tetrahydronaphthalin-2-ylselanyl)-pent-2-en-4-inoat,
3-Methyl-5-(3,5,5,8,8-pentamethyl-5,6,7,8-tetrahydronaphthalin-2-ylselanyl)-pent-2-en-4-in-carbonsäure,
Ethyl-3-methyl-5-(5,5,8,8-tetramethyl-5,6,7,8-tetrahydronaphthalin-2-ylsulfanyl)-pent-2-en-4-inoat,
3-Methyl-5-(5,5,8,8-tetramethyl-5,6,7,8-tetrahydro-naphthalin-2-ylsulfanyl)-pent-2-en-4-in-carbonsäure,
Ethyl-3-methyl-5-(3,5,5,8,8-pentamethyl-5,6,7,8-tetrahydronaphthalin-2-ylsulfanyl)-pent-2-en-4-inoat,
3-Methyl-5-(3,5,5,8,8-pentamethyl-5,6,7,8-tetrahydronaphthalin-2-ylsulfanyl)-pent-2-en-4-in-carbonsäure,
Ethyl-3-methyl-5-(5,5,8,8-tetramethyl-3-propoxy-5,6,7,8-tetrahydro-naphthalin-2-ylselanyl)-pent-2-in-4-inoat,
3-Methyl-5-(5,5,8,8-tetramethyl-3-propoxy-5,6,7,8-tetrahydronaphthalin-2-ylselanyl)-pent-2-en-4-in-carbonsäure,
Ethyl-3-methyl-5-(5,5,8,8-tetramethyl-4-propoxy-5,6,7,8-tetrahydro-naphthalin-2-ylselanyl)-pent-2-en-4-inoat,
3-Methyl-5-(5,5,8,8-tetramethyl-4-propoxy-5,6,7,8-tetrahydronaphthalin-2-ylselanyl)-pent-2-en-4-in-carbonsäure,
Ethyl-5-(4-methoxymethoxy-5,5,8,8-tetramethyl-5,6,7,8-tetrahydro-naphthalm-2-ylselanyl)-3-methyl-pent-2-en-4-inoat,
5-(4-Methoxymethoxy-5,5,8,8-tetramethyl-5,6,7,8-tetrahydronaphthalin-2-ylselanyl)-3-methyl-pent-2-en-4-in-carbonsäure,
Ethyl-5-(3-hydroxy-5,5,8,8-tetramethyl-5,6,7,8-tetrahydronaphthalin-2-ylselanyl)-3-methyl-pent-2-en-4-inoat,
5-(3,5,5,8,8-Pentamethyl-5,6,7,8-tetrahydro-naphthalin-2-ylselanyl)-3-phenyl-pent-2-en-4-in-carbonsäure,
5-(4-Methoxymethoxy-5,5,8,8-tetramethyl-5,6,7,8-tetrahydronaphthalin-2-ylselanyl)-3-phenyl-pent-2-en-4-in-carbonsäure,
Methyl-5-(4-hydroxy-5,5,8,8-tetramethyl-5,6,7,8-tetrahydronaphthalin-2-ylselanyl)-3-phenyl-pent-2-en-4-inoat,
3-Propyl-5-(5,5,8,8-tetramethyl-5,6,7,8-tetrahydro-naphthalin-2-ylselanyl)-pent-2-en-4-in-carbonsäure,
5-(4-Benzyloxy-3-brom-5,5,8,8-tetramethyl-5,6,7,8-tetrahydronaphthalin-2-ylselanyl)-3-methyl-pent-2-en-4-in-carbonsäure,
5-(4-Benzyloxy-5,5,8,8-tetramethyl-5,6,7,8-tetrahydronaphthalin-2-ylselanyl)-3-phenyl-pent-2-en-4-in-carbonsäure,
Ethyl-5-[5-adamant-1-yl-4-(2-methoxy-ethoxymethoxy)-2-methyl-phenylselanyl]-3-methyl-pent-2-en-4-inoat,
5-[5-Adamant-1-yl-4-(2-methoxy-ethoxymethoxy)-2-methylphenylselanyl]-3-methyl-pent-2-en-4-in-carbonsäure,
5-[5-Adamant-1-yl-4-(2-methoxy-ethoxymethoxy)-2-methylphenylselanyl]-3-propyl-pent-2-en-4-in-carbonsäure,
5-[5-Adamant-1-yl-4-(2-methoxy-ethoxymethoxy)-2-methylphenylselanyl]-3-phenyl-pent-2-en-4-in-carbonsäure,
Ethyl-5-(3,5-di-*t*-butyl-2-methoxymethoxy-phenylselanyl)-3-methyl-pent-2-en-4-inoat,
5-(3,5,-Di-*t*-butyl-2-methoxymethoxy-phenylselanyl)-3-methylpent-2-en-4-in-carbonsäure,
Ethyl-3-methyl-5-(5,5,8,8-tetramethyl-3-hexyloxy-5,6,7,8-tetrahydro-naphthalin-2-ylselanyl)-pent-2-en-4-inoat,
3-Methyl-5-(5,5,8,8-tetramethyl-3-hexyloxy-5,6,7,8-tetrahydronaphthalin-2-ylselanyl)-pent-2-en-4-in-carbonsäure,
5-[4-Adamant-1-yl-3-(2-methoxy-ethoxymethoxy)-phenylselanyl]-3-propyl-pent-2-en-4-in-carbonsäure,
Ethyl-5-[2-(adamant-1-yl)-1-methoxyethoxymethoxyphen-5-ylselanyl)-3-methyl-pent-2-en-4-inoat,
Ethyl-5-[4-(2-methoxy-ethoxymethoxy)-5,5,8,8-tetramethyl-5,6,7,8-tetrahydro-naphthalin-2-ylsulfanyl]-3-methyl-pent-2-en-4-inoat,
(Z)-5-[4-(2-Methoxy-ethoxymethoxy)-5,5,8,8-tetramethyl-5,6,7,8-tetrahydro-naphthalin-2-ylsulfanyl]-3-methyl-pent-2-en-4-in-carbonsäure,
(E)-5-[4-(2-Methoxy-ethoxymethoxy)-5,5,8,8-tetramethyl-5,6,7,8-tetrahydro-naphthalin-2-ylsulfanyl]-3-methyl-pent-2-en-4-in-carbonsäure,
Methyl-5-(3,5,5,8,8-pentamethyl-5,6,7,8-Tetrahydronaphthalin-2-ylsulfanyl)-penta-2,4-diinoat,
Ethyl-5-(3-benzyloxy-5,5,8,8-tetramethyl-5,6,7,8-tetrahydronaphthalin-2-ylselanyl)-3-methyl-pent-2-en-4-inoat,
5-(3-Benzyloxy-5,5,8,8-tetramethyl-5,6,7,8-tetrahydronaphthalin-2-ylselanyl)-3-methyl-pent-2-en-4-in-carbonsäure,
Ethyl-5-[3-(2-methoxyethoxymethoxy)-5,5,8,8-tetramethyl-5,6,7,8-tetrahydro-naphthalin-2-ylselanyl]-3-methyl-pent-2-en-4-inoat,
5-[3-(2-Methoxyethoxymethoxy)-5,5,8,8-tetramethyl-5,6,7,8-tetrahydro-naphthalin-2-ylselanyl]-3-methyl-pent-2-en-4-incarbonsäure,
Methyl-5-(3,5,5,8,8-pentamethyl-5,6,7,8-Tetrahydronaphthalin-2-ylselanyl)-penta-2,4-diinoat,
Ethyl-5-(4-benzyloxy-5,5,8,8-tetramethyl-5,6,7,8-tetrahydronaphthalin-2-ylselanyl)-3-methyl-pent-2-en-4-inoat,
Ethyl-5-[3-(5-hydroxy-pentyloxy)-5,5,8,8-tetramethyl-5,6,7,8-tetrahydro-naphthalin-2-ylselanyl]-3-methyl-pent-2-en-4-inoat,
5-(4-Fluor-3-methyl-phenylselanyl)-3-methyl-pent-2-en-4-incarbonsäure,
3-Methyl-5-p-tolylselanyl-pent-2-en-4-in-carbonsäure, und
5-(6-Brom-4,4-dimethyl-thiochroman-8-ylselanyl)-3-methylpent-2-en-4-in-carbonsäure.

12. Verbindungen nach Anspruch 1, **dadurch gekennzeichnet, daß** sie mindestens eine und vorzugsweise alle folgenden Eigenschaften aufweisen:
- R₁ bedeutet eine Gruppe -COR₆;
- X bedeutet Se oder S;
- die Gruppe -X-Y-R1 befindet sich in bezug auf den Substituenten R₃ am aromatischen Ring in para-Stellung;
- R₂ und R₃ bilden gemeinsam mit dem angrenzenden aromatischen Ring einen 5- oder 6-gliedrigen Ring, der gegebenenfalls mit Methylgruppen substituiert und/oder gegebenenfalls durch ein Sauerstoff- oder Schwefelatom unterbrochen ist, oder R₂ oder R₃ bedeutet 1-Adamantyl.

13. Verbindungen nach einem der vorhergehenden Ansprüche zur Verwendung als Arzneimittel.

14. Verbindungen nach Anspruch 13 zur Verwendung als Arzneimittel, das zur Behandlung der folgenden Störungen und Erkrankungen vorgesehen ist: dermatologische Erkrankungen, die mit einer Verhornungsstörung einhergehen, welche mit der Differenzierung und Proliferation zusammenhängt, insbesondere Acne vulgaris, Acne comedonica, polymorphe Acne, Acne rosaceae, nodulocystische Acne, Acne conglobata, Acne senilis, sekundäre Akneformen, wie Acne solaris, Acne medikamentosa oder Acne professionalis; weitere Arten von Keratinisierungsstörungen, insbesondere Ichtyosis, ichtyosisartige Zustände, Darier Krankheit, Palmoplantarkeratosen, Leukoplakien und leukoplakieforme Zustände und Lichen der Haut oder der Schleimhäute (buccalis); weitere dermatologische Erkrankungen, die mit einer Keratinisierungsstörung mit entzündlicher und/oder immunoallergischer Komponente verbunden sind, und insbesondere alle Arten von Psoriasis der Haut, der Schleimhäute oder der Nägel, Psoriasis arthropathica, Atopie der Haut, beispielsweise Ekzeme, oder Atopie der Atemwege oder auch Hypertrophie des Zahnfleisches; verschiedene entzündliche Erkrankungen, die keine Keratinisierungsstörungen aufweisen; alle Proliferationen der Dermis oder Epidermis, die gutartig oder bösartig und gegebenenfalls viralen Ursprungs sein können, beispielsweise Verrucae vulgares, Verrucae planae und Epidermodysplasia verruciformis, Papillomatosis oralis oder florida und Proliferationen, die durch UV-Strahlung hervorgerufen werden können, insbesondere im Falle von Epithelioma basocellulare und spinocellulare; weitere dermatologische Erkrankungen, wie beispielsweise bullöse Dermatosen und Erkrankungen des Kollagens; verschiedene ophthalmologische Störungen, insbesondere Corneopathien; Hautalterung (Behebung oder Bekämpfung), die lichtinduziert oder altersbedingt sein kann, oder Pigmentierungen und aktinische Keratosen oder alle Erkrankungen, die mit der altersbedingten oder aktinischen Hautalterung verbunden sind; Wundmale (Vorbeugung oder Bekämpfung) der epidermalen und/oder dermalen Atrophie, die durch lokal oder systemisch verabreichte Corticosteroide hervorgerufen wird, oder beliebige weitere Formen der Atrophie der Haut, Störungen der Wundheilung oder Streifen (Vorbeugung oder Bekämpfung); Wundheilung (Förderung); Funktionsstörungen der Talgdrüsen, beispielsweise Hyperseborrhoe bei Acne oder Seborrhoe simplex; krebsartige oder präcanceröse Zustände (Behandlung oder Vorbeugung) und insbesondere promyelocytäre Leukämie; entzündliche Erkrankungen, wie Arthritis, beliebige Erkrankungen viralen Ursprungs der Haut oder allgemeine virale Erkrankungen; Alopezie (Vorbeugung oder Behandlung); dermatologische Erkrankungen mit Immunkomponente; Erkrankungen des cardiovasculären Systems, beispielsweise Arteriosklerose, Bluthochdruck, nicht insulinpflichtige Diabetes sowie Adipositas und Hautstörungen, die von einer Exposition gegenüber UV-Strahlung herrühren.

15. Pharmazeutische Zusammensetzung, **dadurch gekennzeichnet, daß** sie in einem pharmazeutisch akzeptablen Träger mindestens eine Verbindung nach einem der Ansprüche 1 bis 12 enthält.

16. Zusammensetzung nach Anspruch 15, **dadurch gekennzeichnet, daß** die Konzentration der Verbindung(en) nach einem der Ansprüche 1 bis 12 im Bereich von 0,001 bis 5 Gew.-%, bezogen auf die gesamte Zusammensetzung, liegt.

17. Kosmetische Zusammensetzung, **dadurch gekennzeichnet, daß** sie in einem kosmetisch akzeptablen Träger mindestens eine Verbindung nach einem der Ansprüche 1 bis 12 enthält.

18. Zusammensetzung nach Anspruch 17, **dadurch gekennzeichnet, daß** die Konzentration der Verbindung(en) nach einem der Ansprüche 1 bis 12 im Bereich von 0,001 bis 3 Gew.-%, bezogen auf die gesamte Zusammensetzung, liegt.

19. Verwendung einer kosmetischen Zusammensetzung nach einem der Ansprüche 17 oder 18 zur Körper- oder Haarpflege.

## Claims

1. Heteroethynylenic compounds, **characterized in that** they correspond to the general formula (I) below: 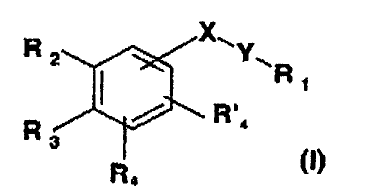 in which:
- R₁ represents:
(i) a -CH₃ radical
(ii) a radical -CH₂-O-R₅
(iii) radical -COR₆
R₅ and R₆ having the meanings given below,
- X represents: O, Se, S(O)n, n being 0, 1 or 2,
- Y represents a divalent radical which has the formula:
a) 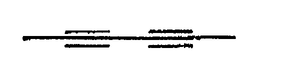
b) 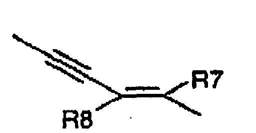 R₇ and R₈ having the meanings given below,
- R₂ and R₃, taken together, form, with the adjacent aromatic ring, a 5- or 6-membered ring optionally substituted with methyl groups and/or optionally interrupted by an oxygen or sulphur atom,
given that R₂ and R₃ independently, which may be identical or different, can represent:
a) a hydrogen atom,
b) a radical chosen from the following radicals
- methyl,
- tert-butyl,
- 1-methylcyclohexyl,
- 1-adamantyl,
c) a radical -OR₉
d) a polyether radical
e) a halogen atom
R₉ having the meaning given below,
it being understood that at least one of the substituents R₂ and R₃ represents (b),
- R₄ represents a hydrogen atom, an alkyl radical of 1 to 6 carbon atoms, a radical OR₁₀, a polyether radical, a radical COR₁₁ or a halogen atom,
R₁₀ and R₁₁ having the meanings given below,
- R'₄ represents a hydrogen atom or a halogen atom,
- R₅ represents a hydrogen atom or an alkyl radical of 1 to 6 carbon atoms,
- R₆ represents:
(i) a hydrogen atom
(ii) an alkyl radical of 1 to 6 carbon atoms
(iii) a radical OR₁₂
R₁₂ having the meaning given below,
(iv) a radical of formula 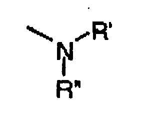 R' and R'' having the meanings given below,
- R₇ and R₈ which may be identical or different, represent a hydrogen atom, an alkyl radical of 1 to 6 carbon atoms or an aryl radical,
- R₉ and R₁₀, which may be identical or different, represent a hydrogen atom, an alkyl radical of 1 to 6 carbon atoms, an optionally substituted aryl radical, an optionally substituted aralkyl radical, a monohydroxyalkyl or polyhydroxyalkyl radical or an acyl radical of 1 to 4 carbon atoms,
- R₁₁ represents an alkyl radical of 1 to 6 carbon atoms, a radical OR₁₃ or a radical 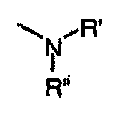 R₁₃, R' and R'' having the meanings given below,
- R₁₂ and R₁₃, which may be identical or different, represent a hydrogen atom, an alkyl radical of 1 to 6 carbon atoms, an aryl radical, an optionally substituted aralkyl radical, a monohydroxyalkyl radical or a polyhydroxyalkyl radical,
- R' and R'', which may be identical or different, represent a hydrogen atom, an alkyl radical of 1 to 6 carbon atoms, a mono- or polyhydroxyalkyl radical, an optionally substituted aryl radical or an amino acid residue, or alternatively, taken together with the nitrogen atom, form a heterocycle,
as well as the salts thereof and the optical and geometrical isomers thereof.

2. Compounds according to Claim 1, **characterized in that** they are in the form of salts of an alkali metal or alkaline earth metal, or alternatively of zinc or of an organic amine.

3. Compounds according to either of Claims 1 and 2, **characterized in that** the alkyl radicals of 1 to 6 carbon atoms are chosen from the group consisting of methyl, ethyl, isopropyl, butyl and tert-butyl radicals.

4. Compounds according to one of the preceding claims, **characterized in that** the monohydroxyalkyl radicals are chosen from the group consisting of 2-hydroxyethyl, 2-hydroxypropyl and 3-hydroxypropyl radicals.

5. Compounds according to one of the preceding claims, **characterized in that** the polyhydroxyalkyl radicals are chosen from the group consisting of 2,3-dihydroxypropyl, 2,3,4-trihydroxybutyl and 2,3,4,5-tetrahydroxypentyl radicals and a pentaerythritol residue.

6. Compounds according to one of the preceding claims, **characterized in that** the aryl radical is a phenyl radical optionally substituted with at least one halogen atom, a hydroxyl radical or a nitro function.

7. Compounds according to one of the preceding claims, **characterized in that** the aralkyl radicals are chosen from the group consisting of benzyl or phenethyl radicals, optionally substituted with at least one halogen atom, a hydroxyl or a nitro function.

8. Compounds according to any one of the preceding claims, **characterized in that** the amino acid residues are chosen from the group consisting of residues derived from one of the 20 amino acids of L or D configuration which constitute mammalian proteins.

9. Compounds according to any one of the preceding claims, **characterized in that** the heterocyclic radicals are chosen from the group consisting of piperidino, morpholino, pyrrolidino and piperazino radicals, optionally substituted in position 4 with a C₁-C₆ alkyl or mono- or polyhydroxyalkyl radical.

10. Compounds according to any one of the preceding claims, **characterized in that** the polyether radicals are chosen from methoxymethyl ether, methoxyethoxymethyl ether and methylthiomethyl ether radicals.

11. Compounds according to Claim 1, **characterized in that** they are taken, alone or as mixtures, from the group consisting of:
Ethyl 3-methyl-5-(5,5,8,8-tetramethyl-5,6,7,8-tetrahydronaphthalen-2-ylselanyl)pent-2-en-4-ynoate
3-Methyl-5-(5,5,8,8-tetramethyl-5,6,7,8-tetrahydronaphthalen-2-ylselanyl)pent-2-en-4-ynoic acid
Ethyl 3-methyl-5-(3,5,5,8,8-pentamethyl-5,6,7,8-tetrahydronaphthalen-2-ylselanyl)pent-2-en-4-ynoate
3-Methyl-5-(3,5,5,8,8-pentamethyl-5,6,7,8-tetrahydronaphthalen-2-ylselanyl)pent-2-en-4-ynoic acid
Ethyl 3-methyl-5-(5,5,8,8-tetramethyl-5,6,7,8-tetrahydronaphthalen-2-ylsulphanyl)pent-2-en-4-ynoate
3-Methl-5-(5,5,8,8-tetramethyl-5,6,7,8-tetrahydronaphthalen-2-ylsulphanyl)pent-2-en-4-ynoic acid
Ethyl 3-methyl-5-(3,5,5,8,8-pentamethyl-5,6,7,8-tetrahydronaphthalen-2-ylsulphanyl)pent-2-en-4-ynoate
3-Methyl-5-(3,5,5,8,8-pentamethyl-5,6,7,8-tetrahydronaphthalen-2-ylsulphanyl)pent-2-en-4-ynoic acid
Ethyl 3-methyl-5-(5,5,8,8-tetramethyl-3-propoxy-5,6,7,8-tetrahydronaphthalen-2-ylselanyl)pent-2-en-4-ynoate
3-Methyl-5-(5,5,8,8-tetramethyl-3-propoxy-5,6,7,8-tetrahydronaphthalen-2-ylselanyl)pent-2-en-4-ynoic acid
Ethyl 3-methyl-5-(5,5,8,8-tetramethyl-4-propoxy-5,6,7,8-tetrahydronaphthalen-2-ylselanyl)pent-2-en-4-ynoate
3-Methyl-5-(5,5,8,8-tetramethyl-4-propoxy-5,6,7,8-tetrahydronaphthalen-2-ylselanyl)pent-2-en-4-ynoic acid
Ethyl 5-(4-methoxymethoxy-5,5,8,8-tetramethyl-5,6,7,8-tetrahydronaphthalen-2-ylselanyl)-3-methylpent-2-en-4-ynoate
5-(4-Methoxymethoxy-5,5,8,8-tetramethyl-5,6,7,8-tetrahydronaphthalen-2-ylselanyl)-3-methylpent-2-en-4-ynoic acid
Ethyl 5-(3-hydroxy-5,5,8,8-tetramethyl-5,6,7,8-tetrahydronaphthalen-2-ylselanyl)-3-methylpent-2-en-4-ynoate
5-(3,5,5,8,8-Pentamethyl-5,6,7,8-tetrahydronaphthalen-2-ylselanyl)-3-phenylpent-2-en-4-ynoic acid
5-(4-Methoxymethoxy-5,5,8,8-tetramethyl-5,6,7,8-tetrahydronaphthalen-2-ylselanyl)-3-phenylpent-2-en-4-ynoic acid
Methyl 5-(4-hydroxy-5,5,8,8-tetramethyl-5,6,7,8-tetrahydronaphthalen-2-ylselanyl)-3-phenylpent-2-en-4-ynoate
3-Propyl-5-(5,5,8,8-tetramethyl-5,6,7,8-tetrahydronaphthalen-2-ylselanyl)pent-2-en-4-ynoic acid
5-(4-Benzyloxy-3-bromo-5,5,8,8-tetramethyl-5,6,7,8-tetrahydronaphthalen-2-ylselanyl)-3-methylpent-2-en-4-ynoic acid
5-(4-Benzyloxy-5,5,8,8-tetramethyl-5,6,7,8-tetrahydronaphthalen-2-ylselanyl)-3-phenylpent-2-en-4-ynoic acid
Ethyl 5-[5-adamantan-1-yl-4-(2-methoxyethoxymethoxy)-2-methylphenylselanyl]-3-methylpent-2-en-4-ynoate
5- [5-Adamantan-1-yl-4-(2-methoxyethoxymethoxy)-2-methylphenylselanyl]-3-methylpent-2-en-4-ynoic acid
5-[5-Adamantan-1-yl-4-(2-methoxyethoxymethoxy)-2-methylphenylselanyl]-3-propylpent-2-en-4-ynoic acid
5-[5-Adamantan-1-yl-4-(2-methoxyethoxymethoxy)-2-methylphenylselanyl]-3-phenylpent-2-en-4-ynoic acid
Ethyl 5-(3,5-di-tert-butyl-2-methoxymethoxyphenylselanyl)-3-methylpent-2-en-4-ynoate
5-(3,5-Di-*tert*-butyl-2-methoxymethoxyphenylselanyl)-3-methylpent-2-en-4-ynoic acid
Ethyl 3-methyl-5-(5,5,8,8-tetramethyl-3-hexyloxy-5,6,7,8-tetrahydronaphthalen-2-ylselanyl)pent-2-en-4-ynoate
3-Methyl-5-(5,5,8,8-tetramethyl-3-hexyloxy-5,6,7,8-tetrahydronaphthalen-2-ylselanyl)pent-2-en-4-ynoic acid
5-[4-Adamantan-1-yl-3-(2-methoxyethoxymethoxy)-phenylselanyl]-3-propylpent-2-en-4-ynoic acid
Ethyl 5-[2-(adamantan-1-yl)-1-methoxyethoxymethoxyphen-5-ylselanyl)-3-methylpent-2-en-4-ynoate
Ethyl 5-[4-(2-methoxyethoxymethoxy)-5,5,8,8-tetramethyl-5,6,7,8-tetrahydronaphthalen-2-ylsulphanyl]-3-methylpent-2-en-4-ynoate
(Z)-5-[4-(2-Methoxyethoxymethoxy)-5,5,8,8-tetramethyl-5,6,7,8-tetrahydronaphthalen-2-ylsulphanyl]-3-methylpent-2-en-4-ynoic acid
(E)-5-[4-(2-Methoxyethoxymethoxy)-5,5,8,8-tetramethyl-5,6,7,8-tetrahydronaphthalen-2-ylsulphanyl]-3-methylpent-2-en-4-ynoic acid
Methyl 5-(3,5,5,8,8-pentamethyl-5,6,7,8-tetrahydronaphthalen-2-ylsulphanyl)penta-2,4-diynoate
Ethyl 5- (3-benzyloxy-5,5,8,8-tetramethyl-5,6,7,8-tetrahydronaphthalen-2-ylselanyl)-3-methylpent-2-en-4-ynoate
5-(3-Benzyloxy-5,5,8,8-tetramethyl-5,6,7,8-tetrahydronaphthalen-2-ylselanyl)-3-methylpent-2-en-4-ynoic acid
Ethyl 5-[3-(2-methoxyethoxymethoxy)-5,5,8,8-tetramethyl-5,6,7,8-tetrahydronaphthalen-2-ylselanyl]-3-methylpent-2-en-4-ynoate
5-[3-(2-Methoxyethoxymethoxy) -5,5,8,8-tetramethyl-5,6,7,8-tetrahydronaphthalen-2-ylselanyl]-3-methylpent-2-en-4-ynoic acid
Methyl 5-(3,5,5,8,8-pentamethyl-5,6,7,8-tetrahydronaphthalen-2-ylselanyl)penta-2,4-diynoate
Ethyl 5-(4-benzyloxy-5,5,8,8-tetramethyl-5,6,7,8-tetrahydronaphthalen-2-ylselanyl)-3-methylpent-2-en-4-ynoate
Ethyl 5-[3-(5-hydroxypentyloxy)-5,5,8,8-tetramethyl-5,6,7,8-tetrahydronaphthalen-2-ylselanyl]-3-methylpent-2-en-4-ynoate
5-(4-Fluoro-3-methylphenylselanyl)-3-methylpent-2-en-4-ynoic acid
3-Methyl-5-p-tolylselanylpent-2-en-4-ynoic acid
5- (6-Bromo-4,4-dimethylthiochroman-8-ylselanyl) -3-methylpent-2-en-4-ynoic acid.

12. Compounds according to Claim 1, **characterized in that** they have at least one, and preferably all, of the following characteristics:
- R₁ represents a radical -COR₆;
- X represents an Se or S radical;
- the group -X-Y-R₁ is in the para position relative to the substituent R₃ on the aromatic ring;
- R₂ and R₃, taken together, form, with the adjacent aromatic ring, a 5- or 6-membered ring optionally substituted with methyl groups and/or optionally interrupted by an oxygen or sulphur atom, or R₂ or R₃ is a 1-adamantyl radical.

13. Compounds according to any one of the preceding claims, for use as medicinal products.

14. Compounds according to Claim 13, for use as medicinal products intended for treating dermatological complaints associated with a keratinization disorder which has a bearing on differentiation and on proliferation, in particular for treating common acne, comedones, polymorphonuclear leukocytes, rosacea, nodulocystic acne, acne conglobata, senile acne and secondary acnes such as solar, medication-related or occupational acne; for treating other types of keratinization disorders, in particular ichthyosis, ichthyosiform states, Darier's disease, palmoplantar keratoderma, leucoplasias and leucoplasiform states, and cutaneous or mucous (buccal) lichen; for treating other dermatological complaints associated with a keratinization disorder with an inflammatory and/or immunoallergic component and, in particular, all forms of psoriasis, whether it is cutaneous, mucous or ungual psoriasis, and even psoriatic rheumatism, or alternatively cutaneous atopy, such as eczema or respiratory atopy or alternatively gingival hypertrophy; the compounds can also be used in certain inflammatory complaints which have no keratinization disorder; for treating all dermal or epidermal proliferations, whether benign or malignant and whether they are of viral origin or otherwise, such as common warts, flat warts and verruciform epidermodysplasia, oral or florid papillomatoses and proliferations which may be induced by ultraviolet radiation, in particular in the case of basocellular and spinocellular epitheliomas; for treating other dermatological disorders such as bullosis and collagen diseases; for treating, certain ophthalmological disorders, in particular corneopathies; for repairing or combating ageing of the skin, whether this is light-induced or chronological ageing, or for reducing actinic keratoses and pigmentations, or any pathologies associated with chronological or actinic ageing; for preventing or curing the stigmata of epidermal and/or dermal atrophy induced by local or systemic corticosteroids, or any other form of cutaneous atrophy, for preventing or treating cicatrization disorders or for preventing or repairing stretch marks; for promoting cicatrization, for combating disorders of sebaceous functioning such as the hyperseborrhoea of acne or simple seborrhoea; in the treatment or prevention of cancerous or precancerous states, more particularly promyelocytic leukaemias; in the treatment of inflammatory complaints such as arthritis, in the treatment of any general or skin complaint of viral origin; in the prevention or treatment of alopecia; in the treatment of dermatological complaints having an immunological component; in the treatment of complaints of the cardiovascular system such as arteriosclerosis, hypertension, non-insulin-dependent diabetes and obesity, in the treatment of skin disorders due to an exposure to UV radiation.

15. Pharmaceutical composition, **characterized in that** it comprises, in a pharmaceutically acceptable support, at least one of the compounds as defined in any one of Claims 1 to 12.

16. Composition according to Claim 15, **characterized in that** the concentration of compound(s) according to one of Claims 1 to 12 is between 0.001% and 5% by weight relative to the whole composition.

17. Cosmetic composition, **characterized in that** it comprises, in a cosmetically acceptable support, at least one of the compounds as defined in any one of Claims 1 to 12.

18. Composition according to Claim 17, **characterized in that** the concentration of compound(s) according to one of Claims 1 to 12 is between 0.001% and 3% by weight relative to the whole composition.

19. Use of a cosmetic composition, as defined in either of Claims 17 and 18, for body or hair hygiene.
